# EUROPEAN PATENT SPECIFICATION

(11) **EP 2 114 413 B1**
(45) Date of publication and mention of the grant of the patent: **22.10.2014**
(21) Application number: 07869361.1
(22) Date of filing: 17.12.2007
(51) Int. Cl.: A61K 31/685, A61K 31/70, A61P 35/00

(54) **THERAPEUTICS FOR TREATING CANCER USING 3-BROMOPYRUVATE**
THERAPEUTISCHE MITTEL ZUR KREBSBEHANDLUNG MIT 3-BROMPYRUVAT
AGENTS THÉRAPEUTIQUES POUR LE TRAITEMENT DU CANCER UTILISANT DU 3-BROMOPYRUVATE

(30) Priority: 18.12.2006 US 870512 P
(43) Date of publication of application: 11.11.2009
(73) Proprietor: The Johns Hopkins University, Baltimore, MD 21218 (US)
(72) Inventor: GESCHWIND, Jean-Francois, Potomac, MD 20854 (US); VALI, Mustafa, Baltimore, MD 21202 (US)
(74) Representative: Helbig, Christian
(86) International application number: PCT/US2007/087740
(87) International publication number: WO 2008/076964

(56) References cited:
- US-A1- 2003 087 961
- SHIN S W ET AL: "Hepatic intra-arterial injection of 3-bromopyruvate in rabbit VX2 tumor", ACTA RADIOLOGICA, INFORMA HEALTHCARE, GB, vol. 47, no. 10, 1 December 2006 (2006-12-01), pages 1036-1041, XP008139875, ISSN: 0284-1851, DOI: DOI:10.1080/02841850600977752
- GESCHWIND J-F H ET AL: "Novel therapy for liver cancer: Direct intraarterial injection of a potent inhibitor of ATP production", CANCER RESEARCH, AMERICAN ASSOCIATION FOR CANCER REREARCH, US, [Online] vol. 62, no. 14, 15 July 2002 (2002-07-15) , pages 3909-3913, XP008139880, ISSN: 0008-5472 Retrieved from the Internet: URL:http://cancerres.aacrjournals.org/>
- MICCOLI L.: 'Intracellular pH governs the subcellular distribution of hexokinase in a glioma cell line' BIOCHEM. J. vol. 313, no. PART 3, 01 February 1996, pages 957 - 962, XP008110028
- HIGASHI T.: 'Relationship between retention index in dual-phase (18)F-FDG PET, and hexokinase-II and glucose transporter-1 expression in pancreatic cancer' J. NUCL. MED. vol. 43, no. 2, February 2002, pages 173 - 180, XP008110054
- MINN H. ET AL.: 'Determination of 2-fluoro-2-deoxy-D-glucose uptake and ATP level for evaluating drug effects in neoplastic cells' RES. EXP. MED. (BERL.) vol. 191, no. 1, 1991, pages 27 - 35, XP008110078
- YAMADA K. ET AL.: 'Factors influencing [F-18] 2-fluoro-2-deoxy-D-glucose (F-18 FDG) uptake in melanoma cells: the role of proliferation rate, viability, glucose transporter expression and hexokinase activity' J. DERMATOL. vol. 32, no. 5, May 2005, pages 316 - 334, XP008110055

## Description

### RELATED APPLICATIONS

This application claims the benefit of U.S. Provisional Application No. 60/870,512, filed December 18, 2006.

### BACKGROUND OF THE INVENTION

One of the most common, profound, and intriguing phenotypes of highly malignant tumors, known for more than six decades, is their ability to metabolize glucose at high rates to synthesize high levels of ATP. Under aerobic conditions more than half of the ATP produced in such tumor cells is derived via glycolysis, in sharp contrast to normal cells, where this value is usually less than 10% and oxidative phosphorylation is the predominant method for ATP generation. Under hypoxic (low oxygen tension) conditions, frequently present within tumors, the already high glycolytic rate may double, allowing the tumor cells to thrive while neighboring normal cells become growth deficient. This is a characteristic of both animal and human tumors including those derived from brain, breast, colon, liver, lung, and stomach. In each tumor type, a close correlation exists among the degree of de-differentiation, growth rate, and glucose metabolism, where the most de-differentiated tumors exhibit the fastest growth and the highest glycolytic rate. In fact, this unique phenotype is used clinically worldwide in Positron Emission Tomography (PET) to detect tumors, assess their degree of malignancy, and in some, cases even predict survival time.

Despite the commonality of the high glycolytic phenotype and its widespread use clinically as a diagnostic tool, it has not been exploited as a major target for arresting or slowing the growth of cancer cells because the underlying molecular basis of the high glycolytic phenotype is not completely characterized. It had long been suspected to involve some type of mitochondrial glycolytic interaction. Recent experiments have demonstrated a requirement for an overexpressed mitochondrially bound form of hexokinase, now identified as Type II hexokinase.

Liver cancer, in particular hepatocellular carcinoma (hepatoma), is one of the most common fatal cancers in the world and soon may reach epidemic levels due to increased incidences of virally-induced hepatitis. Among its numerous victims are not only those with primary tumors that develop directly in the liver but those with secondary tumors that frequently arise in this critical metabolic organ as a result of metastasis from other tissues, e.g., the colon. Unfortunately, traditional treatment options are limited by poor response rates, severe toxicities, and high recurrence rates resulting in a mean survival time of about 6 months. Hepatomas are known to exhibit a high glucose catabolic rate, and where examined carefully, to contain elevated levels of hexokinase bound to their mitochondria. Moreover, in the AS-30D hepatoma, the most extensively studied tumor in this class, it has been shown also that the gene for hexokinase is amplified and that the mRNA levels are markedly elevated. Therapeutic methods directed at inhibition of metabolic activity in hepatoma are limited by the fact that a potent agent directed at any of the metabolic enzymes such as hexokinase in the tumor will also target the patient's metabolic enzymes, resulting in severe toxicity. Thus, less potent, but very specific agents such as antisense molecules, have been used to inhibit tumor metabolic activity.

In recent years, the VX2 tumor, an epidermoid rabbit tumor induced by the Shope papilloma virus, has shown promise as a model system for studying hepatoma. The VX2 tumor grows well when implanted in the rabbit's liver, where it takes on growth properties and a vascularization system similar to many human liver tumors. Thus, it is possible via the method known as transcatheter chemoembolization to deliver anticancer agents directly to the implanted tumor via the hepatic artery. In addition, it has been shown that when delivery is made using certain oils the mixture preferentially localizes in the tumor rather than in the surrounding liver tissue. This is important as it may allow for the targeting of exceptionally potent cancer killing agents directly to the tumor for brief periods of time thus minimizing damage to the surrounding liver tissue and toxicity to the host. The energy metabolism of the VX2 tumor requires further characterization in order to determine to what extent it mimics a rapidly growing hepatoma (e.g. exhibits a high glycolytic phenotype, expresses mitochondrially bound hexokinase, etc.). Shin et al., Act a Radiologica, 47:1036-1041, 2006 disclose a 5-minute infusion of 3-bromopyruvate into the hepatic artery for treating cancer.

### SUMMARY OF THE INVENTION

The present invention provides an effective amount of 3-bromopyruvate for use in treating cancer in a subject in need thereof, wherein the effective amount of 3-bromopyruvate is administered as a continuous intraarterial infusion to the subject for one to four hours. In one embodiment, the use comprises administering an effective amount of a 3-bromopyruvate directly to the arterial blood supply of the cancerous tumor. In certain embodiments, an effective amount of 3-bromopyruvate is administered as a continous intraarterial infusion to the subject in need thereof wherein said infusion may be at least about 1 to about 3 hours.

In one embodiment, an effective dose 3-bromopyruvate administered intraarterially is about 1 mM to 2 mM. In certain embodiments, an effective dose of 3-bromopyruvate administered intraarterially is about 1.75 mM. In another embodiment, an effective dose of 3-bromopyruvate administered intraarterially is about 1 mg/kg to 2 mg/kg. In certain embodiments, an effective dose of 3-bromopyruvate administered intraarterially is about 1.6 mg/kg.

In certain embodiments, the method of treating cancer used in a subject in need thereof comprises administering an effective amount of 3-bromopyruvate as a continuous intraarterial infusion and administering an effective amount of a second agent to the subject. The second agent may be a chemotherapeutic agent, a scavenger compound, or radiation therapy. In certain embodiments, wherein the second agent is a chemotherapeutic agent or a scavenger compound, the second agent may be delivered locally (e.g., intraarterially, intraductally via duct in the breast, nipple, urethra, or in cerebral spinal fluid) or intravenously. Such administration of the second agent may be sequentially or simultaneously with the 3-bromopyruvate. In some embodiments, the second agent may be given prior to or after 3-bromopyruvate. In some embodiments, the 3-bromopyruvate and the second agent may be co-formulated into a pharmaceutical composition. In a further embodiment, the second agent may be a combination of chemotherapeutic agents and/or scavenger compounds. In some embodiments, radiation therapy may be used in combination with 3-bromopyruvate and a chemotherapeutic drug and/or a scavenger compound.

In certain embodiments, the cancer is a solid tumor. In one embodiment, the tumor has a highly glycolytic phenotype. Highly glycolytic tumors may be located in a tissue selected from brain, colon, urogenital, lung, renal, prostate, pancreas, liver, esophagus, stomach, hematopoietic, breast, thymus, testis, ovarian, skin, bone marrow or uterine tissue. In some embodiments, the tumor is a liver tumor.

Other features and advantages of the invention will be apparent from the following detailed description, and from the claims.

### BRIEF DESCRIPTION OF THE FIGURES

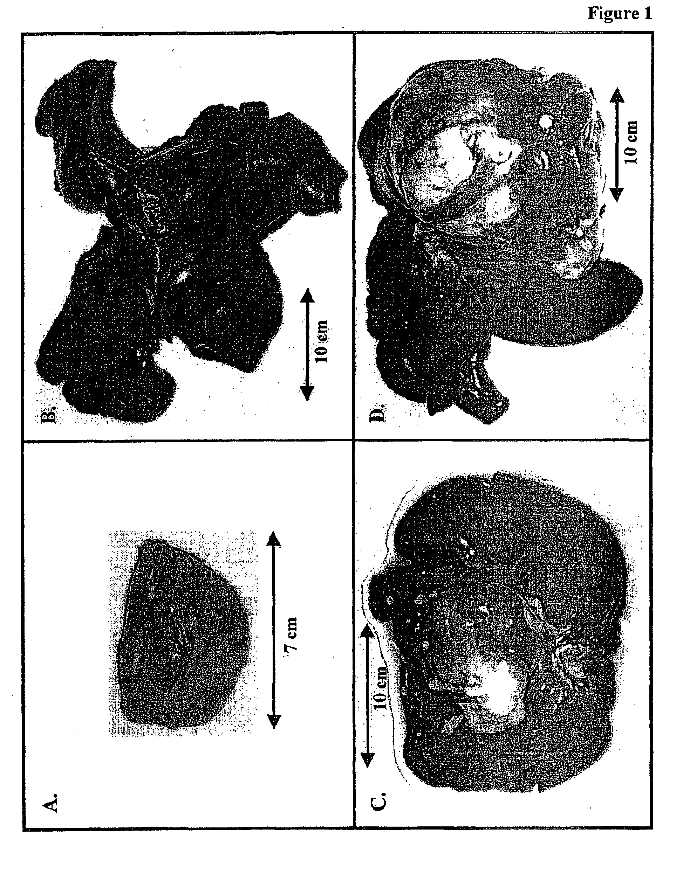

Figure 1 depicts photographs of a VX2 tumor. Figure 1A depicts an excised VX2 tumor after 4 weeks growth in the rabbit hind limb. Figure 1B depicts a control liver isolated from a rabbit of the same age. Figures 1C and 1D depict livers harboring, respectively, VX2 tumors after 4 and 5.7 weeks of implantation.

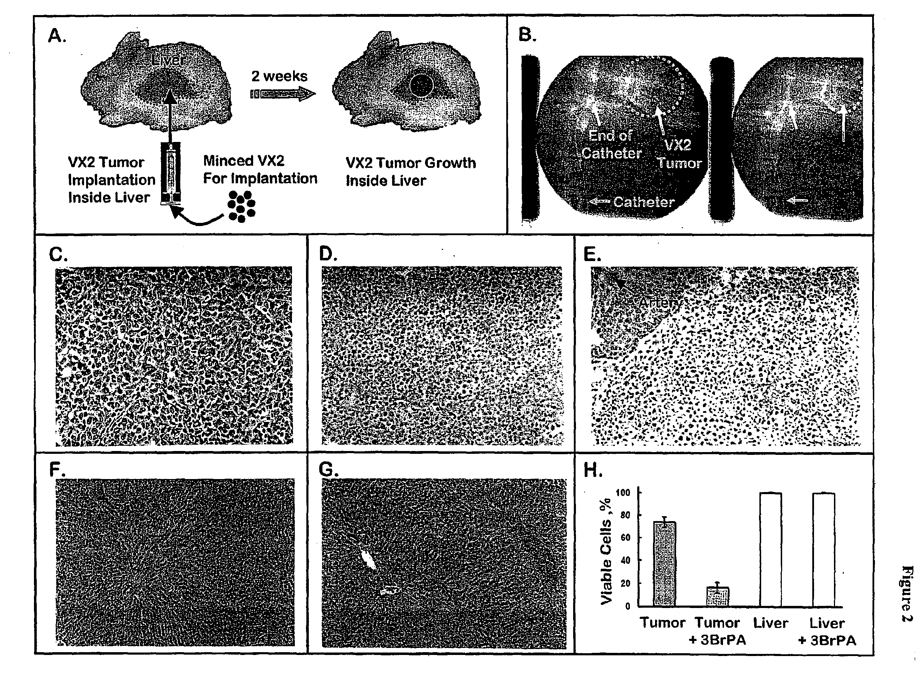

Figure 2 depicts the experimental setup and effect of intraarterial injection of 3-BrPA on liver tumors. Figure 2A depicts a schematic of tumor implantation and growth. Figure 2B depicts two representative hepatic arteriograms. Each shows the hepatic artery leading into a highly vascularized tumor (circled) located within the left lobe. Figure 2C depicts a histological section of a control "untreated" liver implanted tumor isolated 4 days after intraarterial injection of only a saline solution. This section obtained from a region of the tumor located outside the necrotic tumor core shows almost all viable cells. (Magnification = 640x) Figure 2D depicts sections of a liver implanted tumor isolated 4 days after intraarterial injection of 3-BrPA. This section obtained from the same location of the tumor as the control shows no viable cells. (Magnification = 640x) Figure 2E depicts sections from a 3-BrPA treated tumor identical to that in Figure 2D but showing a region near an artery (arrow) where a tiny cluster of cells remains viable. (Magnification = 640x) Figure 2F and 2G depict sections from the liver of a control "untreated" animal and from the liver tissue surrounding an implanted tumor that had been injected intraarterially with 3-BrPA. In both, all cells are viable. (Magnification = 120x). Figure 2H depicts a bar graph summarizing the killing efficacy of intraarterial 3-BrPA on liver tumors. Data are plotted as the mean ± standard deviation. For the liver samples, there was no standard deviation as all cells tested viable.

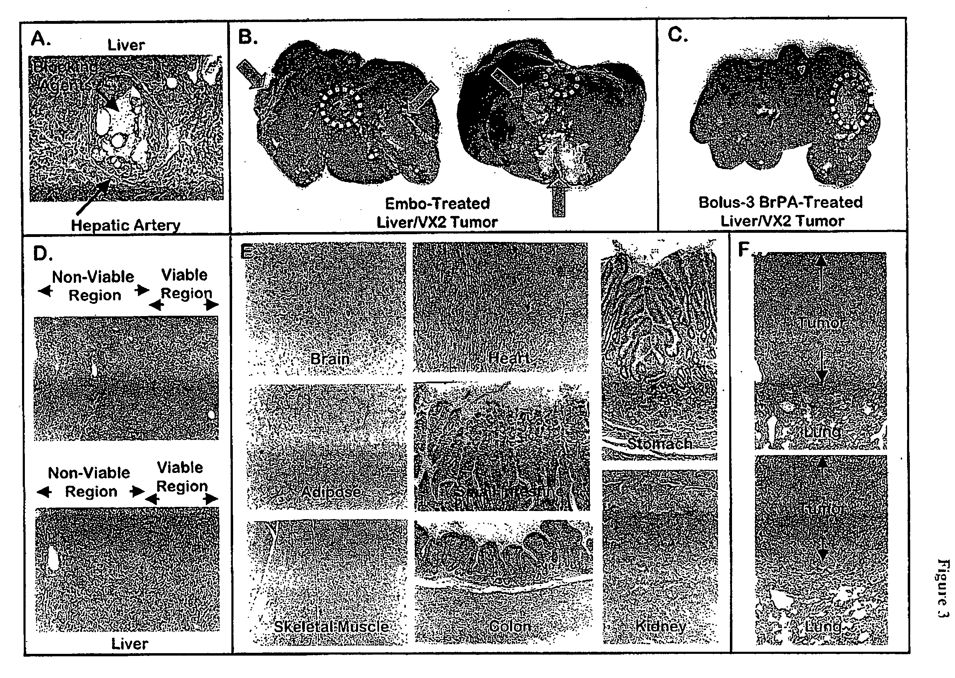

Figure 3 depicts evidence for the benefits of intraarterial therapy for liver cancer using 3-BrPA over current therapy using embolization. Figure 3A depicts a view of the left hepatic artery observed microscopically after injection of embolization material (polyvinyl alcohol) and Ethiodol to block blood flow to the liver (5). (Magnification = 120x). Figure 3B depicts embolized livers harboring VX2 implanted tumors (circles). Arrows indicate damage 4 days after embolization. Figure 3C depicts a liver isolated 4 days after its implanted VX2 tumor (circle) received a single injection of 3-3BrPA. There is no sign of liver damage. Figure 3D depicts histological sections from those regions of livers shown in B that had been affected by embolization. Some tissue has suffered severe damage (non-viable region) and some has remained viable. (Magnification = 120x). Figure 3E depicts sections of 8 tissues from an animal harboring a liver implanted VX2 tumor treated by intraarterial injection of 3-BrPA. All tissues exhibit a normal staining pattern. (Magnification = 120x). Figure 3F depicts sections derived from the same animal showing "metastatic" lung tumors. (Magnification = 120x).

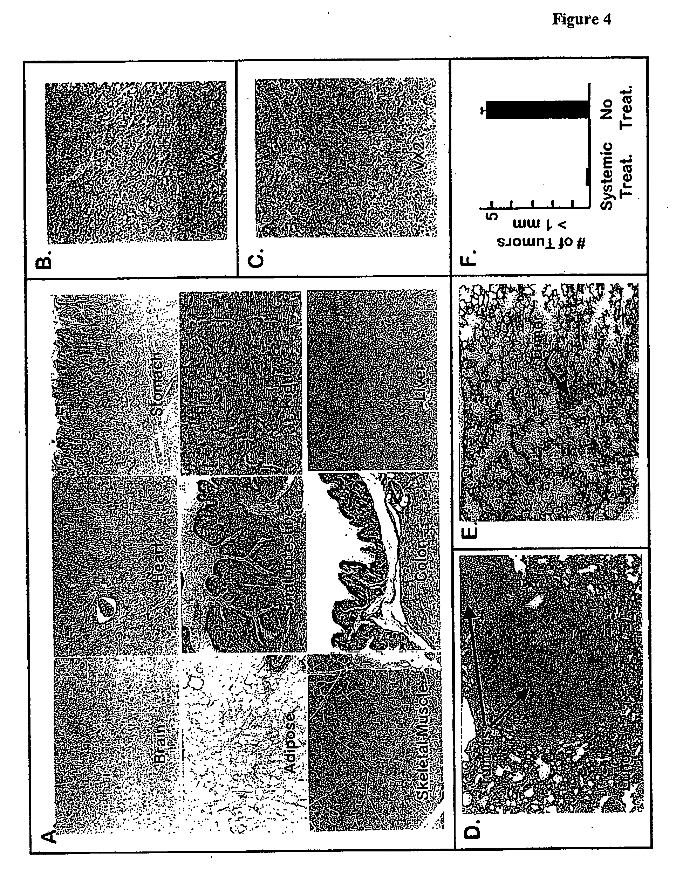

Figure 4 depicts the effect of systemic delivery of 3-BrPA on animals harboring the liver implanted VX2 tumor. Figure 4A depicts histological sections of 9 different tissues isolated 4 days after injecting 3-BrPA (25 ml 0.5 mM) into a marginal ear vein. No damage to these tissues is evident. (Magnification = 120x). Figure 4B depicts a section from a liver implanted VX2 tumor isolated from a control animal not receiving 3-BrPA. Figure 4C depicts a comparable sample from an animal receiving 3-BrPA systemically. Cells in both appear completely viable. (Magnification 120x) Figure 4D depicts a section of lung tissue isolated from an animal in which the liver harbored a VX2 tumor after 14 days of growth. Figure 4E depicts a comparable section isolated from the lung of an identical animal 4 days after receiving a systemic injection of 3-BrPA. (Magnification = 64x) The growth of "metastatic" tumors has been markedly suppressed. Figure 4F depicts a bar graph emphasizing that, of the total number of "metastatic" lung tumors counted (> 27) in comparable histological sections, 5 were greater than 1 mm in diameter in untreated animals harboring a liver implanted VX2 tumor, and none were greater than 1 mm in identical animals that received 3-BrPA systemically. (Animals evaluated = 4).

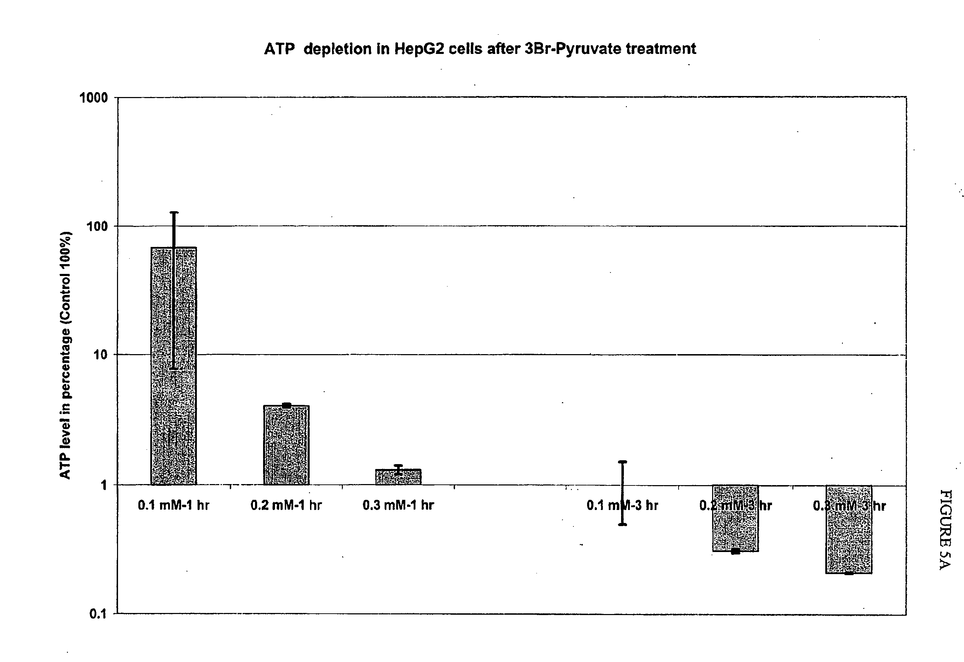

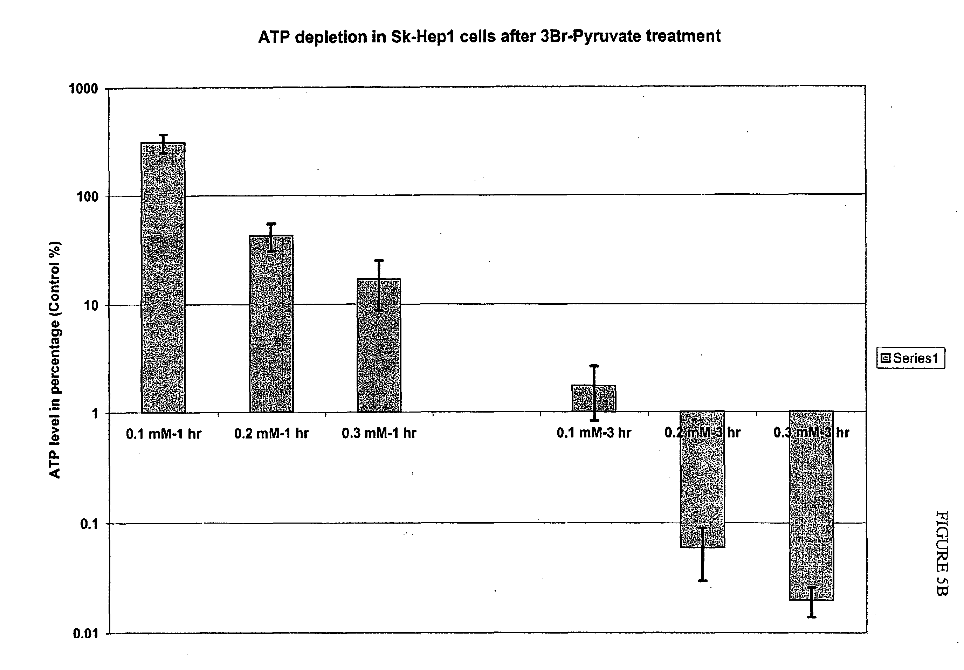

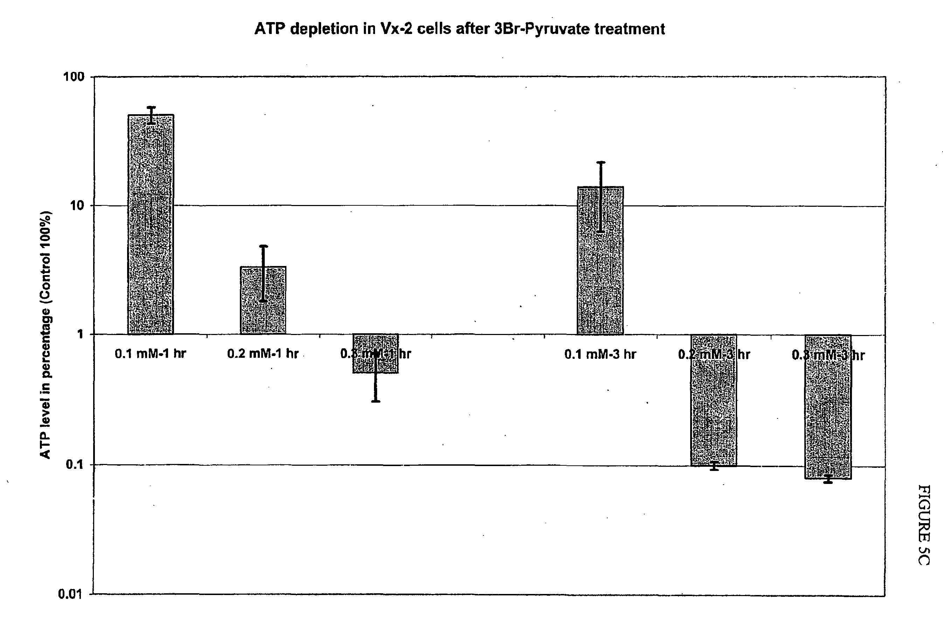

Figure 5 (A-C) are graphs showing ATP depletion in HepG2, Sk-Hep1, and VX2 cells after 3-bromopyruvate treatment.

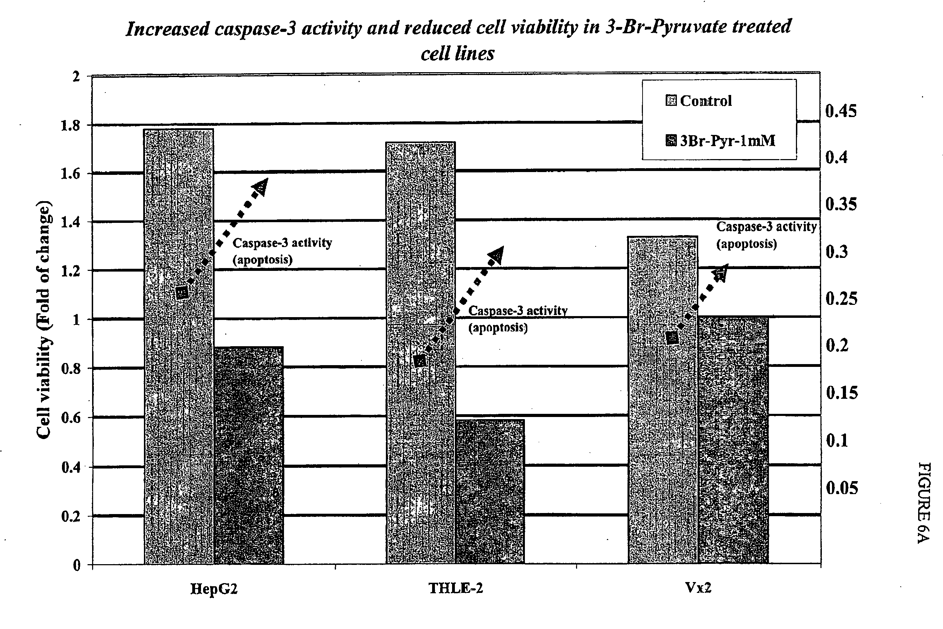

Figures 6 (A) is a graph showing increased caspase-3 activity and reduced cell viability in 3-bromopyruvate (3-Br-Pyruvate) treated cell lines. (B) and (C) are graphs showing 3-bromopyruvate (3Br-Pyr) mediated cytotoxicity after 1 and 3 hours, respectively, by trypan blue staining.

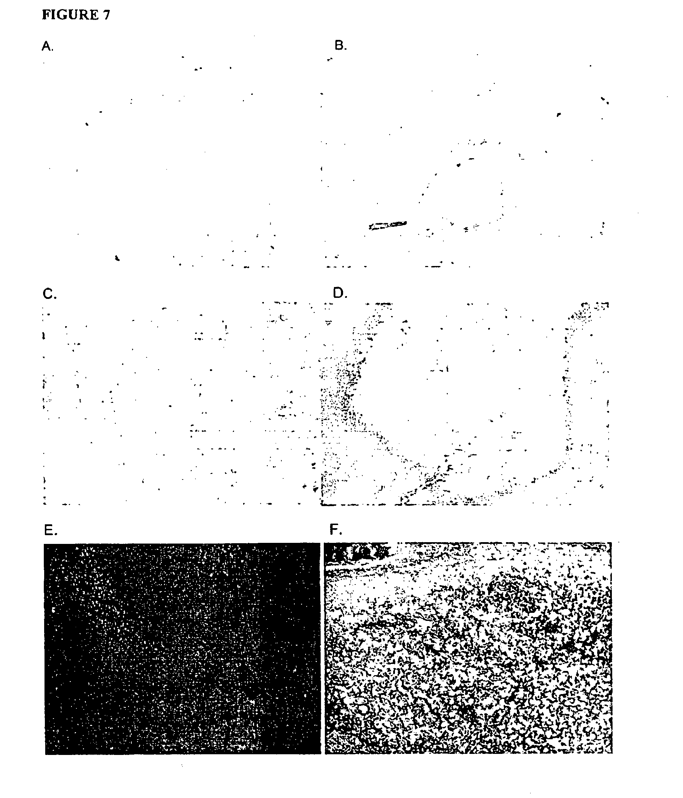

Figure 7 shows histopathology slides. The top four images (A-D) are H&E stained images shown at low and high magnification. The bottom two images are a TUNEL assay of control untreated (E) and treated (F) rabbit showing mostly viable cells in the control animal and complete tumor death in the treated rabbit. The TUNEL assay shows that the mechanism of cell death in the treated animal is apoptotic.

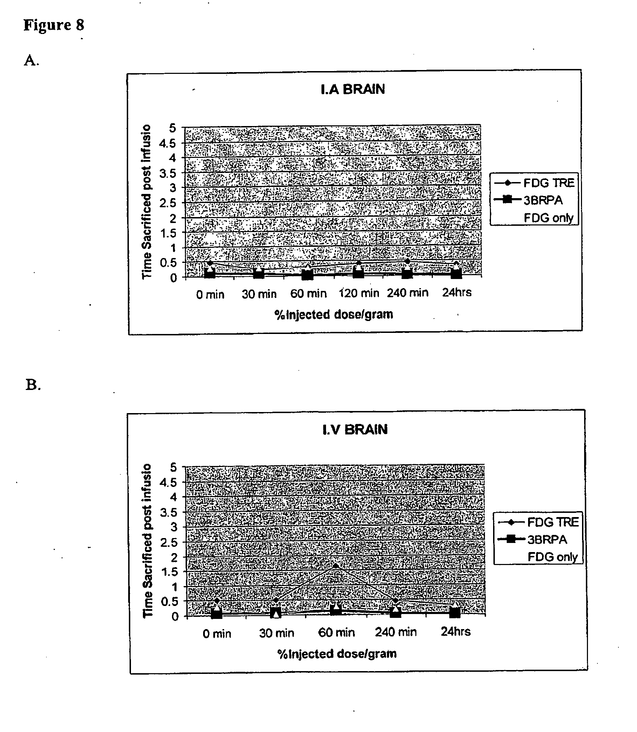

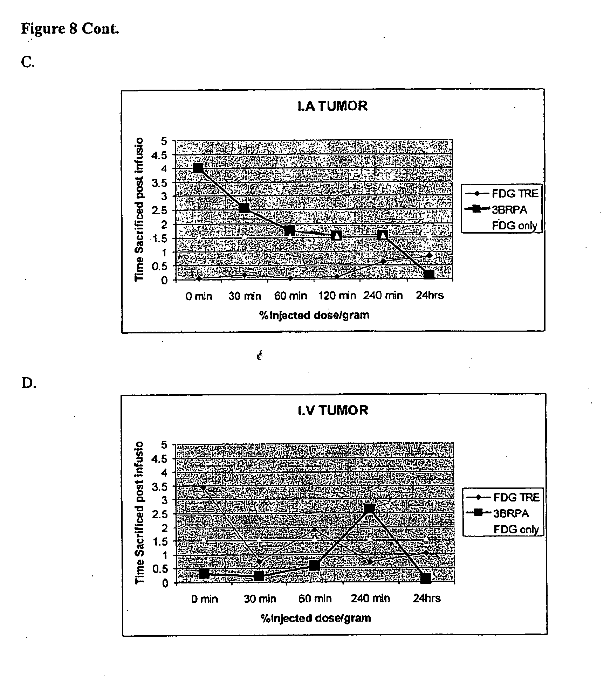

Figure 8 is a series of graphs (A-D) comparing intraarterial infusion (IA) of carbon 14 labeled 3-bromopyruvate compared to intravenous (IV) delivery in brain and tumor. In each graph, FDG tracer (FDG TRE), carbon 14 labeled 3-bromopyruvate (3BRPA), and FDG only are plotted as time scarified post infusion vs. percent injected dose/gram.

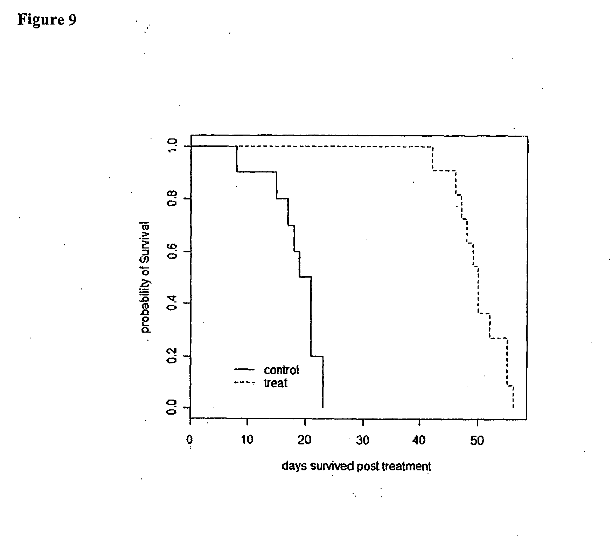

Figure 9 is a graph showing survival of untreated (control) versus 3-bromopyruvate treated animals.

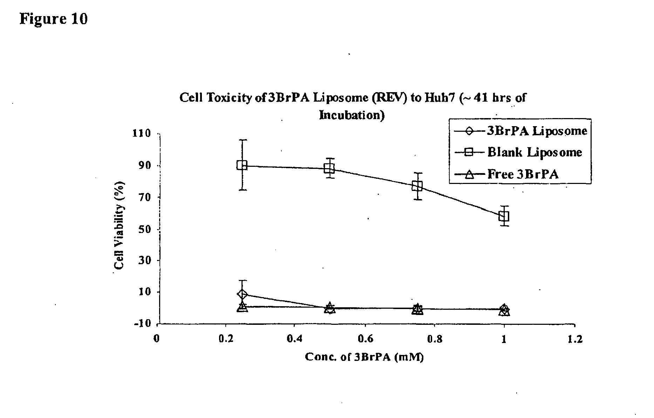

Figure 10 is a graph showing cell toxicity of liposomes containing 3-bromopyruvate on Huh7 cells after approximately 41 hours of incubation.

### DETAILED DESCRIPTION OF THE INVENTION

### 1. Definitions

For convenience, certain terms employed in the specification, examples, and appended claims are collected here. Unless defined otherwise, all technical and scientific terms used herein have the same meaning as commonly understood by one of ordinary skill in the art to which this invention belongs.

The articles "a" and "an" are used herein to refer to one or to more than one (i.e., to at least one) of the grammatical object of the article. By way of example, "an element" means one element or more than one element.

The terms "library" or "combinatorial library" refer to a plurality of molecules, which may be termed "members," synthesized or otherwise prepared from one or more starting materials by employing either the same or different reactants or reaction conditions at each reaction in the library. In general, the members of any library show at least some structural diversity, which often results in chemical and biological diversity. Such structural diversity in preparing libraries of coordination molecules may include, by way of example, metal ion diversity, ligand diversity, solvation diversity or counter-ion diversity. A library may contain any number of members from two different members to about 10⁸ members or more. In certain embodiments, libraries of the present invention have more than about 12, 50 and 90 members. In certain embodiments of the present invention, the starting materials and certain of the reactants are the same, and chemical diversity in such libraries is achieved by varying at least one of the reactants or reaction conditions during the preparation of the library. Combinatorial libraries of the present invention may be prepared in solution or on the solid phase. Further details regarding the libraries of the present invention are described below.

"Modulation" refers to up regulation (i.e., activation or stimulation), down regulation (i.e., inhibition or suppression) of a response, or the two in combination or apart.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrastemal injection and infusion.

A "patient" or "subject" or "host" refers to either a human or non-human animal. The "non-human animals" of the invention comprise any non-human animal that is capable of expressing the subject genes and gene products. Such non-human animals include vertebrates such as rodents, non-human primates, ovines, bovines, ruminants, lagomorphs, porcines, caprines, equines, canines, felines, aves, piscines, etc. In certain embodiments of the invention, the animals are mammals. Exemplary non-human mammals are porcines (e.g., pigs), murines (e.g., rats, mice, and lagomorphs (e.g., rabbits)), and non-human primates (e.g. monkeys and apes).

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

"Pharmaceutically-acceptable salts" refers to the relatively non-toxic, inorganic and organic acid addition salts of compounds.

The phrase "selective inhibitor of ATP production" refers to any compound that is able to specifically modulate the activity of hexokinase or another enzyme that is limiting in the rapid ATP production that provides for the rapid growth of a cancerous tumor. For example, such metabolic pathways include the glycolytic pathway, oxidative phosphorylation pathway, and mitochondrial respiration.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

"Therapeutic agent" or "therapeutic" refers to an agent capable of having a desired biological effect on a host. Chemotherapeutic and genotoxic agents are examples of therapeutic agents that are generally known to be chemical in origin, as opposed to biological, or cause a therapeutic effect by a particular mechanism of action, respectively. Examples of therapeutic agents of biological origin include growth factors, hormones, and cytokines. A variety of therapeutic agents are known in the art and may be identified by their effects. Certain therapeutic agents are capable of regulating red cell proliferation and differentiation. Examples include chemotherapeutic nucleotides, drugs, hormones, nonspecific (non-antibody) proteins, oligonucleotides (e.g., antisense oligonucleotides that bind to a target nucleic acid sequence (e.g., mRNA sequence)), peptides, and peptidomimetics.

"Therapeutic effect" refers to a local or systemic effect in animals, particularly mammals, and more particularly humans caused by a pharmacologically active substance. The term thus means any substance intended for use in the diagnosis, cure, mitigation, treatment or prevention of disease or in the enhancement of desirable physical or mental development and conditions in an animal or human. The phrase "therapeutically-effective amount" means that amount of such a substance that produces some desired local or systemic effect at a reasonable benefit/risk ratio applicable to any treatment. In certain embodiments, a therapeutically effective amount of a compound will depend on its therapeutic index, solubility, and the like. For example, certain compounds discovered by the methods of the present invention may be administered in a sufficient amount to produce a at a reasonable benefit/risk ratio applicable to such treatment.

The phrases "therapeutically-effective amount" and "effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment.

"Treating" a disease in a subject or "treating" a subject having a disease refers to subjecting the subject to a pharmaceutical treatment, e.g., the administration of a drug, such that at least one symptom of the disease is decreased or prevented from worsening.

### 2. General

As described herein, VX2 tumors exhibit a high glycolytic/high hexokinase phenotype, wherein a large fraction of the total cell hexokinase is mitochondrially bound. Thus, the VX2 tumor model may be used in therapeutic screening for inhibitors of tumor metabolic activity, particularly ATP synthesis, that do not damage the surrounding liver tissue and are not toxic to the host. Described herein are the results of a screen for selective inhibitors of VX2 tumor ATP production that may be used in therapeutic methods for treating cancer.

Also described herein is a method for treating cancer comprising direct intraarterial delivery of the compound 3-bromopyruvate (3-BrPA) to liver tumors. 3-bromopyruvate is a strong alkylating agent that abolishes cell ATP production via inhibition of both glycolysis and oxidative phosphorylation. The use of 3-BrPA as a putative therapeutic agent was first suggested by results we obtained using our VX2 tumor inhibitor screening methods. We have shown that this strategy is highly effective, reducing in a single injection the total number of viable cells in liver implanted rabbit tumors to as low as 10% without doing any apparent harm to the animals or their major tissues. Intraarterial injection of 3-bromopyruvate into tumors was further tested to determine optimal methods of administration and dosing regimens.

Further, systemic delivery of 3-BrPA to the same animals bearing the liver implanted tumors, shows no apparent harm to the animals or their major tissues, but suppresses secondary "metastatic" tumors that appear in the lungs. Thus, it is possible with a single, carefully selected known chemical agent, and a combination of intraarterial and systemic delivery methods, to inflict extensive damage on both a primary tumor and a secondary "metastatic" tumor within the same host without doing noticeable harm to the host.

3-Bromopyruvate, as described herein, specifically blocks type II hexokinase and is specific for cancer cells expressing type II hexokinase. Further, the biodistribution of 3-bromopyruvate shows no penetration through the blood brain barrier, when it is administered intraarterially through a prolonged, continuous infusion, e.g., infusion for about 1 hour. The continous infusion of 3-bromopyruvate resulted in complete tumor destruction in aggressive rabbit liver cancer models.

### 3. Inhibitors of ATP Production

Also disclosed are selective inhibitors of ATP production represented in the general formula:

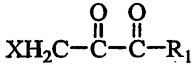

wherein X represents a halide, a sulfonate, a carboxylate, an alkoxide, or an amine oxide. In certain embodiments, X is a halide selected from the group consisting of: fluoride, bromide, chloride, and iodide. In one embodiment, the inhibitor is a 3-halopyruvate. In certain other embodiments, the 3-halopyruvate is selected from the group consisting of: 3-fluoropyruvate, 3-chloropyruvate, 3-bromopyruvate and 3-iodopyruvate. In one embodiment, the 3-halopyruvate is 3-bromopyruvate. In other embodiments, X is a sulfonate is selected from the group consisting of: triflate, mesylate and tosylate. In yet another embodiment, X is an amine oxide is dimethylamine oxide. In certain embodiments R₁ represents OR, H, N(R")₂, C1-C6 alkyl, C6-C12 aryl, C1-C6 heteroalkyl, or a C6-C12 heteroaryl. Independently, in other embodiments, R" represents H, C1-C6 alkyl, or C6-C12 aryl. Independently, in still other embodiments, R represents H, alkali metal, C1-C6 alkyl, C6-C12 aryl or C(O)R'; and R' represents H, C1-C20 alkyl or C6-C12 aryl.

Further disclosed are inhibitors of ATP production represented in general formula:

X-CH2-CO-COOH,

wherein X represents a halide, a sulfonate, a carboxylate, an alkoxide, or an amine oxide. For certain of these compounds, X is a halide selected from the group consisting of: fluoride, bromide, chloride, and iodide. Such inhibitor can be a 3-halopyruvate. The 3-halopyruvate can be selected from the group consisting of: 3-fluoropyruvate, 3-chloropyruvate, 3-bromopyruvate and 3-iodopyruvate. In the invention, however, the 3-halopyruvate is 3-bromopyruvate. Also disclosed are compounds in which X is a sulfonate is selected from the group consisting of triflate, mesylate and tosylate or in which X is an amine oxide is dimethylamine oxide.

### 5. Pharmaceutical Compositions of the Subject Inhibitors

The invention provides pharmaceutical compositions comprising 3-bromopyruvate which are administered as a continuous intraarterial infusion. In one aspect, the present invention provides pharmaceutically acceptable compositions which comprise a therapeutically-effective amount of said compound, formulated together with one or more pharmaceutically acceptable carriers (additives) and/or diluents. In another aspect, certain embodiments, the compound of the invention can be administered as such or in admixtures with pharmaceutically acceptable carriers and can also be administered in conjunction with other chemotherapeutic agents or scavenger compounds. Conjunctive therapy thus includes sequential, simultaneous and separate, or co-administration of the active compound in a way that the therapeutic effects of the first administered one is not entirely disappeared when the subsequent is administered.

The compounds of the present invention are capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared *in situ* in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge et al., *supra*)

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable, antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

In certain embodiments, a formulation of the present invention comprises an excipient selected from the group consisting of cyclodextrins, liposomes, micelle forming agents, e.g., bile acids, and polymeric carriers, e.g., polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions of this invention suitable for the specific parenteral administration as claimed comprise one or more compounds of the invention in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Formulations suitable for continuous intraarterial infusion include liposomes and polymers described herein. In one embodiment, the liposome is about 50 to 100 microns in diameter and is a pegylated stealth liposome with a PEG group attached to the outside of the lipid bilayer so as to avoid causing an immune response and escape degradation by the immune system. In another embodiment, the liposome is about 50 to 100 microns in diameter and is nude liposome without the PEG group which may cause an immune response resulting in an additive antitumor effect. In certain embodiment, the pegylated liposome may be used for systemic administration and the nude liposome may be used for local administration.

In another embodiment, 3-bromopyruvate may be formulated in a polymer that is about 50 to 100 microns in diameter. In one embodiment, 3-bromopyruvate is attached to the polymer. In another embodiment, the granulocyte-macrophage colony stimulating factor (GMCSF) is attached to the polymer. In another embodiment, the 3-bromopyruvate and GMCSF are attached to the polymer. In another embodiment, the polymer comprises 3-bromopyruvate and/or GMCSF. In certain embodiment, inert polymers may also be used as controls.

Exemplary formulations comprising 3-bromopyruvate are determined based on various properties including, but not limited to chemical stability at body temperature, functional efficiency time of release, toxicity and optimal dose.

When the compounds of the present invention are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.

While it is possible for a compound of the present invention to be administered alone, it is preferable to administer the compound as a pharmaceutical formulation (composition).

The compounds according to the invention may be formulated for administration in any convenient way for the claimed use in human or veterinary medicine, by analogy with other pharmaceuticals.

In certain embodiments, the above-described pharmaceutical compositions comprise one or more of the inhibitors, a second chemotherapeutic agent, and optionally a pharmaceutically acceptable carrier.

The term chemotherapeutic agent includes, without limitation, platinum-based agents, such as carboplatin and cisplatin; nitrogen mustard alkylating agents; nitrosourea alkylating agents, such as carmustine (BCNU) and other alkylating agents; antimetabolites, such as methotrexate; purine analog antimetabolites; pyrimidine analog antimetabolites, such as fluorouracil (5-FU) and gemcitabine; hormonal antineoplastics, such as goserelin, leuprolide, and tamoxifen; natural antineoplastics, such as taxanes (e.g., docetaxel and paclitaxel), aldesleukin, interleukin-2, etoposide (VP-16), interferon alfa, and tretinoin (ATRA); antibiotic natural antineoplastics, such as bleomycin, dactinomycin, daunorubicin, doxorubicin, and mitomycin; and vinca alkaloid natural antineoplastics, such as vinblastine and vincristine.

Further, the following additional drugs may also be used in combination with the antineoplastic agent, even if not considered antineoplastic agents themselves: dactinomycin; daunorubicin HCl; docetaxel; doxorubicin HCl; epoetin alfa; etoposide (VP-16); ganciclovir sodium; gentamicin sulfate; interferon alfa; leuprolide acetate; meperidine HCl; methadone HCl; ranitidine HCl; vinblastin sulfate; and zidovudine (AZT). For example, fluorouracil has recently been formulated in conjunction with epinephrine and bovine collagen to form a particularly effective combination.

Still further, the following listing of amino acids, peptides, polypeptides, proteins, polysaccharides, and other large molecules may also be used: interleukins 1 through 18, including mutants and analogues; interferons or cytokines, such as interferons α, β, and y; hormones, such as luteinizing hormone releasing hormone (LHRH) and analogues and, gonadotropin releasing hormone (GnRH); growth factors, such as transforming growth factor-β (TGF-β), fibroblast growth factor (FGF), nerve growth factor (NGF), growth hormone releasing factor (GHRF), epidermal growth factor (EGF), fibroblast growth factor homologous factor (FGFHF), hepatocyte growth factor (HGF), and insulin growth factor (IGF); tumor necrosis factor-α & β (TNF-α & β); invasion inhibiting factor-2 (IIF-2); bone morphogenetic proteins 1-7 (BMP 1-7); somatostatin; thymosin-α-1; γ-globulin; superoxide dismutase (SOD); complement factors; anti-angiogenesis factors; antigenic materials; and pro-drugs.

Preferred chemotherapeutic agents for use with the compositions and methods of treatment described herein include, but are not limited to altretamine, asparaginase, BCG, bleomycin sulfate, busulfan, carboplatin, carmusine, chlorambucil, cisplatin, claladribine, 2-chlorodeoxyadenosine, cyclophosphamide, cytarabine, dacarbazine imidazole carboxamide, dactinomycin, daunorubicin - dunomycin, dexamethosone, doxurubicin, etoposide, floxuridine, fluorouracil, fluoxymesterone, flutamide, fludarabine, goserelin, hydroxyurea, idarubicin HCL, ifosfamide, interferon alfa, interferon alfa 2a, interferon alfa 2b, interfereon alfa n3, irinotecan, leucovorin calcium, leuprolide, levamisole, lomustine, megestrol, melphalan, L-sarcosylin, melphalan hydrochloride, MESNA, mechlorethamine, methotrexate, mitomycin, mitoxantrone, mercaptopurine, paclitaxel, plicamycin, prednisone, procarbazine, streptozocin, tarnoxifen, 6-thioguanine, thiotepa, vinblastine, vincristine and vinorelbine tartrate.

In another preferred embodiment, the composition of the invention may comprise other biologically active substances, preferably a therapeutic drug or pro-drug, for example, other chemotherapeutic agents, scavenger compounds, antibiotics, anti-virals, anti-fungals, anti-inflammatories, vasoconstrictors and anticoagulants, antigens useful for cancer vaccine applications or corresponding pro-drugs.

Exemplary scavenger compounds include, but are not limited to thiol-containing compounds such as glutathione, thiourea, and cysteine; alcohols such as mannitol, substituted phenols; quinones, substituted phenols, aryl amines and nitro compounds.

Various forms of the chemotherapeutic agents and/or other biologically active agents may be used. These include, without limitation, such forms as uncharged molecules, molecular complexes, salts, ethers, esters, amides, and the like, which are biologically activated when implanted, injected or otherwise inserted into the tumor.

### 6. Therapeutic Methods, in which the compounds are used as claimed

The present invention further provides uses in therapeutic methods of treating a cancerous tumor comprising administering to a subject, e.g., a subject in need thereof, an effective amount of 3-bromopyruvate. A subject in need thereof may be a subject, e.g., a human, who has been diagnosed with a tumor or a cancer or a subject who has been treated and has been, e.g., refractory to the previous treatment.

The compounds of the present invention may be used to treat any cancerous tumor. In certain embodiments, the cancerous tumor has a highly glycolytic phenotype. For example, highly glycolytic tumors may be located in a tissue selected from brain, colon, urogenital, lung, renal, prostate, pancreas, liver, esophagus, stomach, hematopoietic, breast, thymus, testis, ovarian, skin, bone marrow or uterine tissue.

The method comprises parenterally administering as a continuous intraarterial infusion an effective amount of 3-bromopyruvate to a subject. In particular embodiments, the method comprises administering an effective amount of 3-bromopyruvate directly to the arterial blood supply of a cancerous tumor in a subject. In one embodiment, the methods comprises administering an effective amount of 3-bromopyruvate directly to the arterial blood supply of the cancerous tumor using a catheter. In embodiments where a catheter is used to administer a subject composition, the insertion of the catheter may be guided or observed by fluoroscopy or other method known in the art by which catheter insertion may be observed and/or guided.

Continuous infusion is effected by means capable of delivering the drug at a specified rate. Continous infusion may be controlled through the use of an external pump permitting adjustment of the rate and duration of infusion. In this case, e.g., 3-bromopyruvate is continuously delivered via a catheter adapted for use in surgery and inserted into the blood vessel, by means of a drug infusor attached to one end of the catheter. The catheter may be one widely used in the field of surgery, such as Teflon catheter, polyethylene catheter or the like. Useful drug infusors are implantable port systems, such as intraarterial infusion pumps that deliver the specified drug intraarterially at a constant rate. For example, a MRI port (Bard Access Systems Inc., Salt Lake City, Utah, USA) may be used. Examples of useful intraarterial infusion pumps are Watkins chronofusor continuous intraarterial infusion pump and Sharp continuous intraarterial infusion pump, MP-22, and Intermate (registered trademark, product of Baxter Healthcare Corporation). Alternatively, the drug may be delivered by drip when such infusors are not usable for one reason or another.

A continuous intraarterial infusion may be administered to a subject in need thereof for 1 hour to 4 hours. In certain embodiments, a continuous intraarterial infusion is administered to a subject in need thereof for at least about 1 hour to about 3 hours. In one embodiment, a continuous intraarterial infusion is administered to the subject in need thereof for about 1 hour. In certain embodiments, the continuous intraarterial infusion is administered to the subject in thereof for 1 hour.

The blood vessels that may be used for infusion are an artery according to the site of the cancer. Examples of arteries usable are the hepatic artery, gastroduodenal artery, subclavian artery, etc. In some embodiments, the methods of treating a cancerous tumor comprise administering an effective amount of 3-bromopyruvate directly to the blood vessels in the liver, head, neck, glands, or bones. For example, blood vessels such as the hepatic, femoral, cerebral, carotid, or vertebral arteries may be infused, injected, chemoembolized, or catheterized to administer the subject compositions to a cancerous tumor. In other embodiments, the methods comprise administering an effective amount of a subject composition directly to the blood vessels in a cancerous tumor in the head, neck, or bones. Such methods are well-known and used in the art. For example, Gobin, Y.P, et al (2001) Radiology 218:724-732 teaches a method for intraarterial chemotherapy for brain tumors. Moser, et al. (2002) Head Neck 24:566-74 reviews the use of intraarterial catheters for chemotherapeutic treatment in head and neck cancer. Wang, M.Q., et al. (2001) J. Vasc. Interv. Radiol. 12:731-7 teaches a method of injecting the femoral arteries as well as a method of chemoembolization in order to treat osteosarcoma. Kato, T., et al. (1996) Cancer Chemother Pharmacol 37(4):289-96 reviews the use of intraarterial infusion of microencapsulated anticancer drugs (chemoembolization) to treat cancerous tumors in the liver, kidney, intrapelvic organs, lung, head and neck, and bones. Hermann, K., et al (2000) Radiology 215:294-9; Kemeny, N.E., (1999) Baillieres Best Pract Res Clin Gastroenterol 13:593-610 describe exemplary methods of intraarterial and embolization methods for treatment of liver cancer.

In general, chemoembolization or direct intraarterial injection therapy utilizing pharmaceutical compositions of the present invention is typically performed in a similar manner, regardless of the site. Briefly, angiography (a road map of the blood vessels), or more specifically in certain embodiments, arteriography, of the area to be embolized may be first performed by injecting radio-opaque contrast through a catheter inserted into an artery (depending on the site to be embolized or injected) as an X-ray is taken. The catheter may be inserted either percutaneously or by surgery. The blood vessel may be then embolized by refluxing pharmaceutical compositions of the present invention through the catheter, until flow is observed to cease. Occlusion may be confirmed by repeating the angiogram. In embodiments where direct injection is used, the blood vessel is then infused with a pharmaceutical composition of the invention in the desired dose.

Embolization therapy generally results in the distribution of compositions containing inhibitors throughout the interstices of the tumor or vascular mass to be treated. The physical bulk of the embolic particles clogging the arterial lumen result in the occlusion of the blood supply. In addition to this effect, the presence of an anti-angiogenic factor(s) prevents the formation of new blood vessels to supply the tumor or vascular mass, enhancing the devitalizing effect of cutting off the blood supply. Direct intraarterial infusion generally results in distribution of compositions containing inhibitors throughout the interstices of the tumor or vascular mass to be treated as well. However, the blood supply is not generally expected to become occluded with this method.

Within one aspect of the present invention, primary and secondary tumors of the liver or other tissues may be treated utilizing embolization or direct intraarterial injection therapy. Briefly, a catheter is inserted via the femoral or brachial artery and advanced into the hepatic artery by steering it through the arterial system under fluoroscopic guidance. The catheter is advanced into the hepatic arterial tree as far as necessary to allow complete blockage of the blood vessels supplying the tumor(s), while sparing as many of the arterial branches supplying normal structures as possible. Ideally this will be a segmental branch of the hepatic artery, but it could be that the entire hepatic artery distal to the origin of the gastroduodenal artery, or even multiple separate arteries, will need to be blocked depending on the extent of tumor and its individual blood supply. Once the desired catheter position is achieved, the artery is embolized by injecting compositions (as described above) through the arterial catheter until flow in the artery to be blocked ceases, preferably even after observation for 5 minutes. Occlusion of the artery may be confirmed by injecting radio-opaque contrast through the catheter and demonstrating by fluoroscopy or X-ray film that the vessel which previously filled with contrast no longer does so. In embodiments where direct injection is used, the artery is infused by injecting compositions (as described above) through the arterial catheter in a desired dose. The same procedure may be repeated with each feeding artery to be occluded.

For use in embolization therapy, compositions of the present invention are preferably non-toxic, thrombogenic, easy to inject down vascular catheters, radio-opaque, rapid and permanent in effect, sterile, and readily available in different shapes or sizes at the time of the procedure. In addition, the compositions preferably result in the slow (ideally, over a period of several weeks to months) release of an inhibitor and/or a second agent. Particularly preferred compositions should have a predictable size of 15-200 microns after being injected into the vascular system. Preferably, they should not clump into larger particles either in solution or once injected. In addition, preferable compositions should not change shape or physical properties.

In another embodiment, the method of administering 3-bromopyruvate to a subject in need thereof comprises chemoembolization. For example, a chemoembolization method may comprise blocking a vessel feeding the cancerous tumor with a composition comprised of a resin-like material mixed with an oil base (e.g., polyvinyl alcohol in Ethiodol) and one or more chemotherapeutic agents. In still other embodiments, the method comprises systemic administration of a subject composition to a subject.

In certain embodiments, administration is conducted by a method that does not involve or comprise embolization, e.g., chemoembolization, or any other method designed to maintain the drug (e.g., 3-bromopyruvate) in the tissue, organ or tumor. In certain embodiments, 3-bromopyruvate is free of oils or other therapeutic composition designed to maintain the drug in the organ, tissue or tumor.

In certain embodiments, the methods of treating a cancerous tumor comprise administering 3-bromopyruvate in conjunction with a second agent to the subject. Such methods in certain embodiments comprise administering pharmaceutical compositions comprising 3-bromopyruvate in conjunction with one or more chemotherapeutic agents and/or scavenger compounds. Conjunctive therapy includes sequential, simultaneous and separate, or co-administration of the active compound in a way that the therapeutic effects of the first administered one is not entirely disappeared when the subsequent is administered. In one embodiment, the second agent is a chemotherapeutic agent. In another embodiment, the second agent is a scavenger compound. In another embodiment, the second agent is radiation therapy. In a further embodiment, radiation therapy may be administered in addition to 3-bromopyruvate and a second agent. In certain embodiments, the second agent may be co-formulated in the separate pharmaceutical composition.

In certain embodiments, 3-bromopyruvate is administered to the subject as a continuous intraarterial infusion and the second agent is administered (e.g., intraarterially, intraductally through a duct in the breast, nipple, urethra, or in cerebral spinal fluid) or intravenously. In further embodiments, the second agent is not administered parenterally, but rather is administered systemically.

In certain embodiments, 3-bromopyruvate may be administered to a subject who is refractory or resistant to certain chemotherapeutic agents and/or radiation. Intraarterial administration of 3-bromopyruvate alone or in combination with a second agent, e.g., a chemotherapeutic agent delivered locally (e.g., intraarterially, intraductally through a duct in the breast, nipple, urethra, or in cerebral spinal fluid) or intravenously may be used to reverse the refractory tumors. In some embodiments, intraarterial administration of 3-bromopyruvate alone or in combination with a second agent, e.g., a chemotherapeutic agent, may be further combined with radiation therapy to treat refractory tumors.

In most embodiments, the subject pharmaceutical compositions will incorporate the substance or substances to be delivered in an amount sufficient to deliver to a patient a therapeutically effective amount of an incorporated therapeutic agent or other material as part of a prophylactic or therapeutic treatment. The desired concentration of active compound in the particle will depend on absorption, inactivation, and excretion rates of the drug as well as the delivery rate of the compound. It is to be noted that dosage values may also vary with the severity of the condition to be alleviated. It is to be further understood that for any particular subject, specific dosage regimens should be adjusted over time according to the individual need and the professional judgment of the person administering or supervising the administration of the compositions. Typically, dosing will be determined using techniques known to one skilled in the art.

For 3-bromopyruvate, an intraarterial dose may range from at least about 1 mM to about 2 mM. In certain embodiments, an intraarterial dose may be about 1.5 mM to about 2 mM. In some embodiments, an intraarterial dose may be about 1.75 mM.

Dosage may be based on the amount of the composition per kg body weight of the patient. For example, a range of amounts of compositions are contemplated, including about 0.001, 0.01, 0.1, 0.5, 1, 10, 15, 20, 25, 50 mg or more of such compositions per kg body weight of the patient. Other amounts will be known to those of skill in the art and readily determined.

In certain embodiments, the dosage of the subject compounds will generally be in the range of about 0.001 mg to about 10 mg per kg body weight, specifically in the range of about 0.1 mg to about 10 mg per kg, and more specifically in the range of about 0.1 mg to about 1 mg per kg. In one embodiment, the dosage is in the range of about 0.3 mg to about 0.6 mg per kg. In one embodiment, the dosage is in the range of about 0.4 mg to about 0.5 mg per kg.

For 3-bromopyruvate, the dosage for intraarterial administration may range from about 1 mg/kg to about 2 mg/kg. In certain embodiments, the dosage for intraarterial administration is about 1.5 mg/kg to about 2 mg/kg. In some embodiments, the dosage for intraarterial administration is about 1.6 mg/kg. In intravenous administration, the dosage may range from about 12 mg/kg to about 20 mg/kg. In certain embodiments, the dosage for intravenous administration may range from about 15 to 18 mg/kg. In some embodiments, the dosage for intravenous administration is about 16.8 mg/kg.

Alternatively, the dosage of the subject invention may be determined by reference to the plasma concentrations of the composition. For example, the maximum plasma concentration (Cmax) and the area under the plasma concentration-time curve from time 0 to infinity (AUC (0-4)) may be used. Dosages for the present invention include those that produce the above values for Cmax and AUC (0-4) and other dosages resulting in larger or smaller values for those parameters.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above.

If desired, the effective daily dose of the active compound may be administered as two, three, four, five, six or more sub-doses administered separately at appropriate intervals throughout the day, optionally, in unit dosage forms.

The precise time of administration and amount of any particular compound that will yield the most effective treatment in a given patient will depend upon the activity, pharmacokinetics, and bioavailability of a particular compound, physiological condition of the patient (including age, sex, disease type and stage, general physical condition, responsiveness to a given dosage and type of medication), route of administration, and the like. The guidelines presented herein may be used to optimize the treatment, e.g., determining the optimum time and/or amount of administration, which will require no more than routine experimentation consisting of monitoring the subject and adjusting the dosage and/or timing.

While the subject is being treated, the health of the patient may be monitored by measuring one or more of the relevant indices at predetermined times during a 24-hour period. Treatment, including supplement, amounts, times of administration and formulation, may be optimized according to the results of such monitoring. The patient may be periodically reevaluated to determine the extent of improvement by measuring the same parameters, the first such reevaluation typically occurring at the end of four weeks from the onset of therapy, and subsequent reevaluations occurring every four to eight weeks during therapy and then every three months thereafter. Therapy may continue for several months or even years, with a minimum of one month being a typical length of therapy for humans. Adjustments to the amount(s) of agent administered and possibly to the time of administration may be made based on these reevaluations.

Treatment may be initiated with smaller dosages which are less than the optimum dose of the compound. Thereafter, the dosage may be increased by small increments until the optimum therapeutic effect is attained. In addition, the combined use 3-bromopyruvate and a second agent, e.g. another chemotherapeutic agent or a scavenger compound, may reduce the required dosage for any individual component because the onset and duration of effect of the different components may be complimentary.

As described above, 3-bromopyruvate, e.g., an intraarterial infusion of 3-bromopyruvate, may be administered in combination with radiation therapy. An optimized dose of radiation therapy may be given to a subject as a daily dose. Optimized daily doses of radiation therapy may be from about 0.25 to 0.5 Gy, about 0.5 to 1.0 Gy, about 1.0 to 1.5 Gy, about 1.5 to 2.0 Gy, about 2.0 to 2.5 Gy, and about 2.5 to 3.0 Gy. An exemplary daily dose may be from about 2.0 to 3.0 Gy. A higher dose of radiation may be administered if a tumor is resistant to lower doses of radiation. High doses of radiation may reach 4 Gy. Further, the total dose of radiation administered over the course of treatment may range from about 50 to 200 Gy. In an exemplary embodiment, the total dose of radiation administered over the course of treatment ranges from about 50 to 80 Gy. In certain embodiments, a dose of radiation may be given over a time interval of 1, 2, 3, 4, or 5 minutes, wherein the amount of time is dependent on the dose rate of the radiation source.

In certain embodiments, a daily dose of optimized radiation may be administered 4 or 5 days a week, for approximately 4 to 8 weeks. In an alternate embodiment, a daily dose of optimized radiation may be administered daily seven days a week, for approximately 4 to 8 weeks. In certain embodiments, a daily dose of radiation may be given a single dose. Alternately, a daily dose of radiation may given as a plurality of doses. In a further embodiment, the optimized dose of radiation may be a higher dose of radiation than can be tolerated by the patient on a daily base. As such, high doses of radiation may be administered to a patient, but in less frequent dosing regimen.

The types of radiation that may be used in cancer treatment are well known in the art and include electron beams, high-energy photons from a linear accelerator or from radioactive sources such as cobalt or cesium, protons, and neutrons. An exemplary ionizing radiation is an x-ray radiation.

Methods to administer radiation are well known in the art. Exemplary methods include, but are not limited to, external beam radiation, internal beam radiation, and radiopharmaceuticals. In external beam radiation, a linear accelerator is used to deliver high-energy x-rays to the area of the body affected by cancer. Since the source of radiation originates outside of the body, external beam radiation can be used to treat large areas of the body with a uniform dose of radiation. Internal radiation therapy, also known as brachytherapy, involves delivery of a high dose of radiation to a specific site in the body. The two main types of internal radiation therapy include interstitial radiation, wherein a source of radiation is placed in the effected tissue, and intracavity radiation, wherein the source of radiation is placed in an internal body cavity a short distance from the affected area. Radioactive material may also be delivered to tumor cells by attachment to tumor-specific antibodies. The radioactive material used in internal radiation therapy is typically contained in a small capsule, pellet, wire, tube, or implant. In contrast, radiopharmaceuticals are unsealed sources of radiation that may be given orally, intravenously or directly into a body cavity.

Radiation therapy may also include sterotactic surgery or sterotactic radiation therapy, wherein a precise amount of radiation can be delivered to a small tumor area using a linear accelerator or gamma knife and three dimensional conformal radiation therapy (3DCRT), which is a computer assisted therapy to map the location of the tumor prior to radiation treatment.

Toxicity and therapeutic efficacy of subject compounds may be determined by standard pharmaceutical procedures in cell cultures or experimental animals, e.g., for determining the LD₅₀ and the ED₅₀. Compositions that exhibit large therapeutic indices are preferred. Although compounds that exhibit toxic side effects may be used, care should be taken to design a delivery system that targets the compounds to the desired site in order to reduce side effects.

The data obtained from the cell culture assays and animal studies may be used in formulating a range of dosage for use in humans. The dosage of any supplement, or alternatively of any components therein, lies preferably within a range of circulating concentrations that include the ED₅₀ with little or no toxicity. The dosage may vary within this range depending upon the dosage form employed and the route of administration utilized. For agents of the present invention, the therapeutically effective dose may be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information may be used to more accurately determine useful doses in humans. Levels in plasma may be measured, for example, by high performance liquid chromatography.

### EXEMPLIFICATION

The invention now being generally described, it will be more readily understood by reference to the following examples which are included merely for purposes of illustration of certain aspects and embodiments of the present invention. The examples contain reference examples, i.e. examples relating to intravenous administration.

Examples 1 through 11 describe work done with VX2 tumors to characterize the energy metabolism of the VX2 tumor, and to screen inhibitors of the metabolism in the VX2 tumor. Examples 12 through 20 describe work done with VX2 tumors implanted in a rabbit host, including the administration of the inhibitors identified in Examples 10 and 11 to the host in order to study the effect of inhibitors in treating the tumor. Example 21 describes assays measuring ATP depletion and cell viability following treatment with 3-bromopyruvate. Examples 22 and 23 describe work done with VX2 tumors and the HCC cell line HepG2 to characterize the specificity of 3-bromopyruvate as an anti-glycolytic agent that binds type II hexokinase. Examples 24 through 26 describe the optimization of dosing regimens for intraarterial and intravenous administration and the biodistribution profile of 3-bromopyruvate. Example 27 describes survival analysis of treated animals compared to untreated animals. Example 28 describes experiments using liposomes comprising 3-bromopyruvate.

### Example 1: Materials for VX2 Tumor Experiments.

New Zealand white rabbits weighing 3.5-4.2 kg were obtained from Robinson Services Inc. The VX2 tumor, normally grown in the hind limb of these animals, was obtained locally from Dr. John Hilton, Department of Oncology, Johns Hopkins University School of Medicine. AS-30D hepatoma cells, an established line, are maintained by Min Gyu Lee in the inventors' laboratory. This is done by growth and passage of the cells in the peritoneal cavity of female Sprague Dawley rats (Charles River Breeding Laboratories). The following agents were obtained from Sigma: D-glucose, 2-deoxyglucose, 2-fluoro-2-deoxyglucose, 6-fluoro-6-deoxyglucose, 3-O-methylglucose, 5-thio-D-glucose-6-phosphate, L-glucose, D-xylose, D-lyxose, 3-bromopyruvic acid, ATP, ADP, NADP+, D-mannitol, Hepes, succinate, oligomycin, and bovine albumin. The lactic acid kit containing lactic dehydrogenase, NAD+, hydrazine, and a glycine buffer, pH 9.2 was obtained also from Sigma. NaPi, KPi, and sucrose were obtained from J.T. Baker, and the Coomassie dye binding agent from Pierce. Glucose-6-phosphate dehydrogenase was obtained from Roche Molecular Biochemicals, and both the DMEM tissue culture medium and trypan blue were from Life Technologies Gibco BRL. The Clark oxygen electrode was purchased from Yellow Springs Instruments.

### Example 2: Processing the VX2 tumor for biochemical analyses.

In one set of studies the VX2 tumors, which had grown in the hind limb of New Zealand white rabbits for 4 weeks, were excised, cut into 1g pieces (ca. 10 mm x 10 mm x 10 mm) with a razor blade, and then subjected to assays described below for monitoring both glycolytic and hexokinase activities. In a second set of studies VX2 tumors, which had grown in the hind limb of a New Zealand white rabbit for about 2 weeks, were excised, broken into small chunks (< 0.1 g), and then implanted into the livers of a number of other rabbits. Following implantation, VX2 tumors rapidly developed in the livers of each animal. They were excised at different times ranging from 2 to 5.7 weeks and also subjected to the assays described below for monitoring glycolytic and hexokinase activities, as well as an assay for monitoring mitochondrial respiration. In all cases the exterior surface of the tumor was shaved to remove any remaining normal tissue. In addition, special care was taken to assure that only the viable portion of the tumor located near or on the surface was removed for analyses. The preparation of the animals for surgery, and the surgical and implantation procedures, have been described. These procedures were approved by the Animal Care and Use Committee at the Johns Hopkins University School of Medicine and conducted according to their guidelines.

### Example 3: Assay for glycolytic activity in the VX2 Tumor.

Glycolysis was assayed by monitoring the formation of lactic acid following the addition of glucose to a medium containing VX2 tumor slices. Specifically, freshly excised tumors were washed 3 times at 4 °C in 20 ml Chance Hess Medium containing 6.2 mM KCl, 154 mM NaCl, and 11 mM NaPi, pH 7.4. Slices, 1 g each, were then prior incubated for 30 min in a Lab-Line incubator-shaker at 37 °C in 2 ml of the same medium while shaking at 50 rpm. Glucose was then added to give a final concentration of 6 mM, after which the incubation/shaking process was continued with 0.2 ml samples being removed every 30 min for up to 2 h. Then, samples were removed for analysis at 2 additional time points indicated in the Figure. These samples were subjected to centrifugation at 14,000 rpm in an Eppendorf Centrifuge (Model 5415C). Then, aliquots of the supernatant, 0.01 ml, were removed from each sample, diluted with 0.03 ml of water and subjected to lactic acid determination using the kit supplied by Sigma. The latter contains, in addition to lactic dehydrogenase and NAD+, hydrazine, and glycine buffer, pH 9.2. The mixture was incubated for 30 min at 25 °C after which the absorbance due to formation of NADH was determined at 340 nm using a Gilford Model 260 spectrophotometer. To assess the elevation of the glycolytic activity of the VX2 tumor over that of liver tissue, parallel studies were always carried out in an identical manner with 1 g liver slices derived from the host liver. Finally, in those cases where agents were tested for their capacity to inhibit glycolysis, these were introduced into the system at the prior incubation step with all subsequent steps being run in parallel with those described above for the VX2 tumor or liver alone.

### Example 4: Preparation of subcellular fractions for hexokinase and mitochondrial respiration assays.

The freshly isolated liver or VX2 tumor tissues (15-20 g wet weight) was rinsed in 3 volumes of H-medium (210 mM D-mannitol, 70 mM sucrose, 2 mM Hepes, and 0.05% bovine albumin, pH 7.4) at 4 °C and minced with a razor blade as finely as possible. A 30% (V/V) suspension was made using ice-cold H-medium in a Potter-Elvehjem glass homogenizer (55 ml capacity), and homogenization was achieved by applying 4 complete up and down strokes through the suspension with a rotating (∼ 400 rpm), serrated, teflon pestle attached to a motor. To remove cell debris and nuclei, the resultant homogenate was diluted to twice the initial volume and centrifuged at 630 x g for 8 minutes at 4 °C in a Sorvall RC-2B centrifuge using a GSA rotor. The supernatant was removed and then centrifuged at 6,800 g for 15 min under the same conditions. The resultant supernatant was saved and referred to here as the cytosolic faction. The pellet was resuspended in the initial volume of H-medium and centrifuged twice at 9,800 x g for 15 min at 4 °C. The washed pellet represents the mitochondrial fraction.

### Example 5: Assay for hexokinase activity.

The assay coupled the glucose-6-phosphate formed in the hexokinase reaction to the glucose-6-phosphate dehydrogenase reaction. Here, NADP+, oxidizes glucose-6-phosphate to a g-lactone while becoming reduced to NADPH, thus allowing the formation of the latter to be monitored spectrophotometrically at 340 nm. The final reaction mixture contained the following ingredients in a total volume of 1 ml at 25 °C: 25 mM triethanolamine, pH 7.6, 15 mM MgCl2, 1 mM dithiothreitol, 0.45 mM NaCN, 0.005 mg/ml oligomycin, 0.014 mM DAPP [P1,P5-Di (adenosine-5') pentaphosphate], 5 mM ATP, 3.3 units glucose-6-phosphate dehydrogenase, 1 mM NADP+, 0.1-0.3 mg cytosolic or mitochondrial fraction, and concentrations of glucose as indicated in the figure legends. Glucose was used to initiate the reaction.

### Example 6: Assay for mitochondrial respiration.

Oxygen consumption rates were measured polarographically using a Clark oxygen electrode inserted into a 2.5 ml chamber equipped with a magnetic stirrer. The electrode was connected to a chart recorder calibrated between 0 and 100% saturation with atmospheric oxygen at 25 °C. The loss of oxygen was monitored in a 2.1 ml system at 25 °C containing 1 mg mitochondria, 0.5 mM EDTA, 2.0 mM Hepes, 220 mM D-mannitol, 70 mM sucrose, 2.5 mM KPi, 2.6 mM MgCl2, and 0.5 mg/ml bovine albumin, and, where indicated, 7.8 mM succinate (respiratory substrate), and 0.24 mM ADP (ATP synthesis substrate).

### Example 7: AS-30D hepatoma cells: culture conditions, and assessment of cell viability.

AS-30D hepatoma cells grown in the peritoneal cavity of Sprague Dawley female rats (see Materials) were adapted to grow in tissue culture in Dulbecco's modified Eagle's medium containing 10% fetal bovine serum. Cells were maintained at 37 °C in a humidified atmosphere containing 5% CO2, and counted in a Neubauer chamber after trypan blue dye addition (cell/dye volume = 1/1) by visualization under a Nikon inverted microscope.

### Example 8. Description of the VX2 tumor prior to and after liver implantation.

Photographs of VX2 tumors representative of those used in this study are presented in Figure 1. When excised from the hind limb of the New Zealand white rabbit (donor) at 4 weeks the tumor is about 10 g in weight, flesh colored with some surface vascularization, and without any obvious signs of necrosis (Figure 1A). When small chunks (< 0.1 g) are implanted in the liver of a rabbit of similar size and age the tumor grows rapidly attaining a weight as high as 25 g in a 4 week period while retaining its solid, flesh colored features (Figure 1C). Once again, there are no obvious signs of necrosis although an increased surface vascularization is apparent. At 5.7 weeks (Figure 1D), the liver implanted VX2 tumor has become highly vascularized on its surface and more than doubled in size in the intervening 1.7 weeks. Much of the increased weight is due to fluid that has accumulated within the core of the tumor where it has become almost completely necrotic and taken on a mush-like texture. At this stage only the tissue near the surface of the tumor remains viable and can be used for biochemical studies. Tumors were not carried beyond 5.7 weeks.

### Example 9: Glycolytic capacity and hexokinase activity of the VX2 tumor prior to and after liver implantation.

To investigate glycolytic capacity, lactic acid production by VX2 tumors isolated from the rabbit hind limb and rabbit liver was measured. Slices of tumor were incubated in the presence of 6 mM glucose for the times indicated and then assayed for lactic acid. Liver slices from the animals from which the tumors were obtained were subjected to the same assay.

Slices of the VX2 tumor isolated after 4 weeks of growth in the hind limb of a New Zealand white rabbit exhibit a glycolytic rate that is 7.4 fold higher than that observed for normal liver tissue derived from the same animal. Significantly, this enhanced glycolytic rate of the VX2 tumor relative to liver tissue is retained when small chunks of the tumor (< 0.1 g) are implanted in the liver of another rabbit of similar size and age. The glycolytic rate of tumor slices derived from the liver implanted VX2 tumor after 4 weeks of growth is 8.3 fold higher than the glycolytic rate obtained with liver slices obtained from the same liver in which the tumor implant had been made. This high glycolytic rate of the liver implanted VX2 tumor remained relatively constant through 5.7 weeks of growth.

The activity of hexokinase per mg protein in liver and VX2 tumor tissues was also measured. The hexokinase activity is markedly elevated (∼ 9.5 fold) in the liver implanted VX2 tumor relative to the activity of this enzyme in the surrounding liver tissue. The mean ± standard error are 13.7 ± 2.5 (liver) and 131 ± 10.1 (tumor). In addition, the subcellular distribution of this activity in the tumor (∼70% in the mitochondrial fraction and ∼30% in the cytosol) differs markedly from that in the surrounding liver tissue (∼ 20% in the mitochondrial fraction and 80% in the cytosol). For liver, the mean ± standard error in % of total cellular distribution is 85.3 ± 2.6 (cytosol) and 13.7 ± 2.5 (mitochondria). For the VX2 tumors these values are 22.6 ± 6 (cytosol) and 77.4 ± 6 (mitochondria). The % of the total starting proptein recovered in the mitochondrial fraction (∼ 20-21%) was nearly the same for liver and tumor. Finally, the Km of the tumor hexokinase(s) for glucose is very low (0.11 mM, mitochondrial fraction and 0.19 mM, cytosolic fraction, mean value of 3 experiments) reflecting a high apparent affinity of the isoform(s) for glucose. This is in sharp contrast to liver where the Km for glucose of the major hexokinase isoform involved, i.e., glucokinase, is at least 5 mM in most reported studies. The mean ± standard error is 0.18 ± 0.015 mM (cytosolic fraction) and 0.13 ± 0.02 mM (mitochondrial fraction).

These studies show that the VX2 tumor, although of non-hepatic origin, exhibits glucose metabolic properties characteristic of many rapidly growing hepatomas, the biochemical hallmarks of which are a high glycolytic/high hexokinase phenotype, and binding of the hexokinase (low Km for glucose) to the mitochondrial fraction.

### Example 10: Screening of inhibitors of the glycolytic capacity of the isolated liver implanted VX2 tumor.

A limited screen was carried out to identify inhibitors of the glycolytic capacity of the VX2 tumor with the purpose of selecting agents that might prove effective in arresting tumor cell growth. The screen included the following 9 compounds: 2-deoxyglucose (2DOG), 2-fluoro-2-deoxyglucose, 6-fluoro-6-deoxyglucose, 3-O-methyl glucose, 5-thio-D-glucose-6-phosphate, L-glucose, D-xylose, D-lyxose, and 3-bromopyruvic acid (3BrPA). The screen was conducted by incubating VX2 tumor slices in a medium containing 6 mM glucose with 6 mM of each of these agents at 37 °C for 5 h. The use of 6 mM glucose in the medium was used to mimic the maximal amount of glucose that might be in the blood in a real *in vivo* situation. All glucose analogs or other sugars tested under these conditions proved to be ineffective as inhibitors of VX2 tumor glycolysis indicating the inability of these sugars to act as effective glycolytic inhibitors under the conditions specified. In sharp contrast, the pyruvate analog (3BrPA) almost completely inhibited glycolysis.

Two of the 9 compounds screened were studied in more detail. One was 2DOG because under certain conditions it is known to inhibit glycolysis when it is phosphorylated by hexokinase to 2DOG-6-P which cannot be further metabolized. The other compound tested in more detail was 3BrPA because of its effectiveness as a glycolytic inhibitor in the preliminary screen.

To compare the inhibitory effects of 2DOG and 3BrPA on the glycolytic capacity of the isolated liver implanted VX2 tumor, various concentrations of 2DOG and 3BrPA were prior incubated for 30 min at 37 oC with 1 g VX2 tumor slices and glycolysis was monitored.

2DOG can inhibit glycolysis catalyzed by VX2 tumor slices provided it is used at concentrations of glucose much higher than those normally found in the blood, and provided it is prior incubated with the tumor slices in the absence of glucose. In particular, half maximal inhibition of glycolysis requires about 15 mM 2DOG whereas maximal inhibition (70%) requires almost 50 mM. 3BrPA, however, is a more effective inhibitor than 2DOG as it induces half maximal inhibition of the glycolytic activity of the VX2 tumor slices at a concentration of only 2.4 mM and complete inhibition at about 15 mM.

Additional experiments were undertaken to determine whether 3BrPA is also an inhibitor of the mitochondrial hexokinase of the VX2 tumor, as this enzyme is known to be markedly elevated in rapidly growing hepatomas, and where examined carefully, to be required for maintenance of the high glycolytic rate. 5 mM 3BrPA inhibits completely glucose initiation of the hexokinase reaction in a system containing among other ingredients VX2 tumor mitochondria, ATP, glucose-6-phosphate dehydrogenase, and NADP+. Although complete inhibition of hexokinase activity is achieved at a concentration of only 5 mM 3BrPA, a concentration near 15 mM is necessary to completely inhibit glycolysis. This is likely due to the fact that the former assay was conducted on a cell free extract whereas the latter was conducted on intact tumor tissue. Finally, in addition to these findings, was the very important discovery that 3BrPA (1.2 mM) also completely inhibits mitochondrial respiration, i.e., both the basal rate of respiration catalyzed by the respiratory substrate succinate, and the ADP stimulated rate of respiration normally associated with ATP synthesis by oxidative phosphorylation.

### Example 11: Comparison of the relative capacities of 2DOG and 3BrPA to kill hepatoma cells expressing the high glycolyticlhigh hexokinase phenotype.

The studies described above using the VX2 tumor model for liver cancer predict that 2DOG and 3BrPA may have the capacity to kill hepatoma cells expressing the high glycolytic/high hexokinase phenotype. For this reason, both agents were tested for their capacity to inhibit the growth of AS-30D hepatoma cells, an established rat cell line known to exhibit a high glycolytic rate and to contain elevated levels of mitochondrial bound hexokinase. Significantly, comparison of control hepatoma cells with those treated 12 h with 20 mM 2DOG or with 5 mM 3BrPA show that both 2DOG and 3BrPA have the capacity to induce cell death, with 3BrPA killing all cells in the population and 2DOG killing about 80%. Cell killing was assessed in these experiments by counting those cells in which trypan blue had entered.

### Example 12: VX2 Tumor Implantation.

The rabbit VX2 tumor was selected for implantation in the liver because of the similarities of its blood supply to that of human hepatomas. Other attributes of this tumor include rapid tumor growth, development of a sizable tumor that can be readily identified by x-ray imaging (fluoroscopy), and a biochemical phenotype characteristic of advanced stage tumors, i.e., high glycolysis and elevated levels of mitochondrial bound hexokinase. In addition, the rabbit is large enough that selective manipulation of catheters in the hepatic artery from the common femoral artery for delivery of agents is possible. Adult New Zealand white rabbits (32 total, Robinson Services, Inc.) weighing 3.5-4.2 kg were used. Studies with these animals were approved by the Johns Hopkins University Animal Care and Use Committee and carried out according to their guidelines. For successful implantation of the VX2 tumor into the liver, it was first grown for 2 weeks on the hind leg of a carrier rabbit. Each carrier rabbit was used to supply tumor cells for implantation into the left lobe of the liver of 2 separate rabbits. All animals, carriers and recipients, were anesthetized with a mixture of acepromazine (2.5 mg/kg) and ketamine hydrochloride (44 mg/kg) administered intramuscularly. Intravenous access was gained via a marginal ear vein and sodium pentothal was given intravenously to maintain anesthesia. The VX2 tumor was then excised from the carrier rabbit and placed in Hanks solution. Chunks of the tumor were minced in the same solution. Then, the abdomens of the recipient rabbits were shaved and prepped with betadine after which a midline subxyphoid incision was made. The anterior surface of the liver was exposed and tumor cells (0.1-0.2 ml) from the minced donor tumor were directly implanted onto the left lobe of the liver using the outer cannula of a 21-gauge angiocatheter. This method allows the growth of a single solitary, well-demarcated tumor in the liver of each recipient rabbit. The abdomen was closed in 2 layers. Proper aseptic technique was rigorously observed during each implantation. After surgery, animals were returned to their cages, kept warm with blankets, and monitored in the animal laboratory under the direct supervision of a physician or a technician until they recovered from anesthesia. Buprenorphine (0.01 mg) was administered for analgesia when the animals were in pain or showed physical distress. The tumors were allowed to grow for another 14 days at which time they reached an ellipsoidal shape with dimension of approximately 1.5 x 1.8 x 2.5 cm.

### Example 13: Preparation of 3-BrPA solutions.

The solutions of 3-bromopyruvate (3-BrPA, Sigma Chemical Co., St. Louis, MO) were prepared in phosphate-buffered saline (PBS). After adjusting the pH to 7.0 with NaOH the solutions were sterilized via Millipore's Millexâ GV 0.22 mm filter unit and used immediately. Freshly made solutions were used in all studies reported here,

### Example 14: Intraarterial Injection of 3-bromopyruvate.

Administration of anesthesia, intravenous access and sodium pentothal anesthesia were carried out as described in Example 12. Transcatheter hepatic artery injection of 3-BrPA was performed under fluoroscopy. The animals were brought to the angiography suite and intubated using a size 3.0 mm endotracheal tube (Mallinkrodt Medical, St. Louis, MO) but not ventilated. Surgical cut-down was performed to gain access into the right common femoral artery, after which a 3 French sheath (Cook Inc., Bloomington, IN) was placed. A specially manufactured 2 French catheter with a tip in the shape of a hockey-stick (JB1 catheter, Cook Inc., Bloomington, IN) was manipulated into the celiac axis after which a celiac arteriogram was performed in order to delineate the blood supply to the liver and confirm the location of the tumor. The tumor could readily be visualized as a region of hypervascular blush located on the left side of the liver near the gastric fundus. The left hepatic artery, which usually provides most of the blood flow to the tumor, was selectively catheterized via the common hepatic artery. When necessary, a steerable guidewire (0.010-0.014 inches Transend wire, Boston Scientific MediTech, Natick, MA) was used to help select the left hepatic artery. After having adequately positioned the catheter within the left hepatic artery, the 3-BrPA solution was infused directly into the artery. The animals were monitored after the procedure and given analgesics when they showed signs of physical distress.

### Example 15: Embolization.

This procedure was performed in a manner similar to the technique described above for 3-BrPA. However, instead of using 3-BrPA, a mixture of Ethiodol and embolic material (polyvinyl alcohol, Target Incorporated, Fremont, CA) was injected into the left hepatic artery. The procedure was considered successful when forward flow was no longer demonstrated within the left hepatic artery. In addition, an intense tumor stain was identified in each case suggesting a successful embolization procedure.

### Example 16: Histopathology and Statistical Analysis.

Normal tissues and tumors were fixed in 10% formalin, sliced at 5 mM intervals for gross examination, and then embedded completely in paraffin after which 4 m sections were stained with hemotoxylin and eosin. Tumor viability was estimated by visual inspection and expressed as a percentage of viable tumor area for each slice. The overall percentage of viable tumor in each rabbit was calculated.

The mean fractions of tumor necrosis + SD were compared using the unpaired Student t test for between group comparisons. Differences were considered statistically significant for p<0.05.

### Example 17: Direct Intraarterial Injection of 3-BrPA into Liver Implanted VX2 Tumors Selectively Inhibits the Viability of Cells Therein without Altering the Viability of Surrounding Liver Tissue.

To test our hypothesis that direct intraarterial injection of a potent inhibitor of cell ATP production (3-BrPA) may selectively inhibit the viability of cells within the tumor, we employed the established VX2 tumor model for reasons described above. Small chunks of a donor VX2 tumor were minced, surgically implanted in the livers of 6 rabbits/experiment, and allowed to grow for 14 days (Figure 2A). At this time, the single well- delineated tumor that developed in each liver exhibited a high degree of arterial vascularization due to the onset of angiogenesis. After fasting the animals for 24 hours and administering anesthesia, a catheter was carefully inserted into the femoral artery and guided by fluoroscopy into the hepatic artery to a position near the tumor site (Figure 2B). Then, a single bolus injection of 3-BrPA was delivered in about 2 min directly into the artery. Animals treated identically, but not receiving 3-BrPA served as controls. Optimal results were obtained by delivering 25 ml 0.5 mM 3-BrPA, waiting 4 days, and then excising and subjecting each tumor, and the surrounding liver tissue, to histological analysis.

The results obtained from this novel approach proved to be quite dramatic. Compared to control "untreated" tumors, where representative sections (7 slides/tumor) obtained outside the central core region revealed nearly 100 % viable cells (Figure 2C), similarly located sections obtained from tumors treated with 3-BrPA (Figure 2D) contained almost all non-viable cells (nearly 100 % necrosis). Viable tumor cells were detected only in small areas near arteries feeding the tumors (Figure 2E), and at the tumor periphery where sinusoidal blood is available. This may reflect more active mitochondria in these oxygen rich environments that are not completely debilitated at the concentrations of 3-BrPA used. Significantly, no damage occurred to liver tissue surrounding tumors that had been treated with 3-BrPA (Figures 2F and 2G).

These results, reproduced in a number of experiments, were subjected to statistical evaluation. Tumors untreated with 3-BrPA (controls) contain 74 ± 5% viable cells in the entire population (Figure 2H, 1 st column). The remaining cells, located within the hypoxic tumor core, have already become non-viable, a common feature of rapidly growing solid tumors. Treatment with a single intraarterial injection of 3-BrPA decreases the number of viable cells to 16 ± 5% (Figure 2H, 2nd column), thus increasing the total number of non-viable cells in the population to 84 ± 5% (P< 0.05). The maximal number of non-viable cells observed in any one experiment was 90%. In sharp contrast, the surrounding liver tissue remained completely viable in all cases examined (Figure 2H, 3rd and 4th columns).

The portal veins, sinusoids, and bile ducts remained completely intact, with the only apparent damage occurring occasionally in the peribiliary arteriolar complexes at much higher concentrations of 3-BrPA (5 mM). These and the above findings suggest that most of the 3-BrPA injected directly into the tumor remained therein, and if any leakage occurred, most was neutralized by natural reducing agents (e.g., glutathione) present in the surrounding tissue.

### Example 18: Conventional Therapy for Advanced Stage Liver Tumors Employing Embolization Results in Significant Damage to Surrounding Liver Tissue.

We next inquired how this new strategy compares with the approach called "embolization" or "chemoembolization" which is currently used to treat advanced stage liver cancer in humans. Embolization involves blocking the hepatic artery feeding the tumor with a resin-like material mixed with an oil base (e.g., polyvinyl alcohol in Ethiodol), thus depriving the tumor of its oxygen and nutrient sources. Chemoembolization refers to the same procedure but with the inclusion of one or more anticancer agents. Using the same rabbit model, we found that embolization alone of the hepatic artery (Figure 3A) leading into the VX2 tumor causes such severe damage to the surrounding liver tissue that it is visually evident (Figure 3B). This is in sharp contrast to the normally appearing liver tissue surrounding VX2 tumors that were not embolized but instead were subjected to direct intraarterial injection of 3-BrPA (Figure 3C). These findings were further substantiated by histological analyses that revealed extensive non-viable liver tissue surrounding tumors treated by embolization (Figure 3D), as opposed to only viable tissue surrounding tumors treated by intraarterial injection of 3-BrPA (Figures 3F and 3G).

### Example 19: The Major Tissues of Animals Bearing 3-BrPA-Treated Liver Tumors Show No Apparent Damage.

Despite the promising results obtained in support of direct intraarterial injection of 3-BrPA as a therapy for liver cancer, the possibility still existed that 3-BrPA may be damaging other organs. For this reason, 9 major tissues were isolated from animals harboring liver implanted VX2 tumors 4 days after receiving a single intraarterial injection of 3-BrPA. In no case was there evidence for damage to these tissues (Figures 3E and 3F). However, the unexpected discovery was made that secondary tumors had developed in the lungs (Figure 3F), a finding observed also in animals bearing liver implanted tumors that had not been treated with 3-BrPA. As this was a consistent finding (n = 6 animals), and because there was no evidence of such tumors in the 8 other major tissues examined, these distant lesions are most likely the result of metastatic spread of the VX2 tumor from the liver to the lung.

### Example 20: Systemic Delivery of 3-BrPA Has No Noticeable Effect on the Animals' Health or Behavior and No Effect on Liver Implanted VX2 Tumors, but Does Markedly Suppress the Growth of Metastatic Lung Nodules.

Finally, it was important to examine the effect of 3-BrPA when delivered systemically (i.e., via the general circulation) on both animal toxicity and its capacity to damage liver implanted tumors. Following delivery of 3-BrPA (25 ml, 0.5 mM) via a marginal ear vein, rabbits that had been harboring liver implanted VX2 tumors for 14 days exhibited normal behavior and, upon sacrifice, histological examination of 9 major tissues revealed no obvious damage (Figure 4A). Moreover, there was no killing effect on liver implanted VX2 tumors (Figures 4B & 4C) as we had observed earlier following direct intraarterial delivery of 3-BrPA (Figures 4C & 4D), thus adding further support for this targeted approach as a preferred therapy for liver cancer. However, in sharp contrast to the failure of systemic delivery of 3-BrPA to be therapeutic for liver implanted VX2 tumors (Figures 4B & 4C), it was found to be therapeutic for secondary tumors that had developed in the lungs. Interestingly, animals bearing the liver implanted VX2 tumors developed numerous "metastatic" nodules in their lungs, the largest of which were several mm in diameter (Figure 4D). Most striking in these animals following systemic treatment with 3-BrPA was the finding of only very small tumors (Figure 4E), and the almost complete disappearance of those with a diameter greater than 1 mm (Figure 4F).

### Example 21: 3-bromopyruvate treatment depletes ATP and increases cell toxicity

ATP levels were measured in various cell lines, e.g., HepG2, Sk-Hep1, and Vx-2 after 3-bromopyruvate treatment. In each case, cells were treated with increasing concentration of 3-bromopyruvate (0.1 mM, 0.2 mM, and 0.3 mM) for 1 and 3 hours beofre measuing ATP levels in the cell. Figures 5 (A-C) show the ATP depletion observed following 3-bromopyruvate treatment in HepG2, Sk-Hep1, and Vx-2 cell lines, respectively.

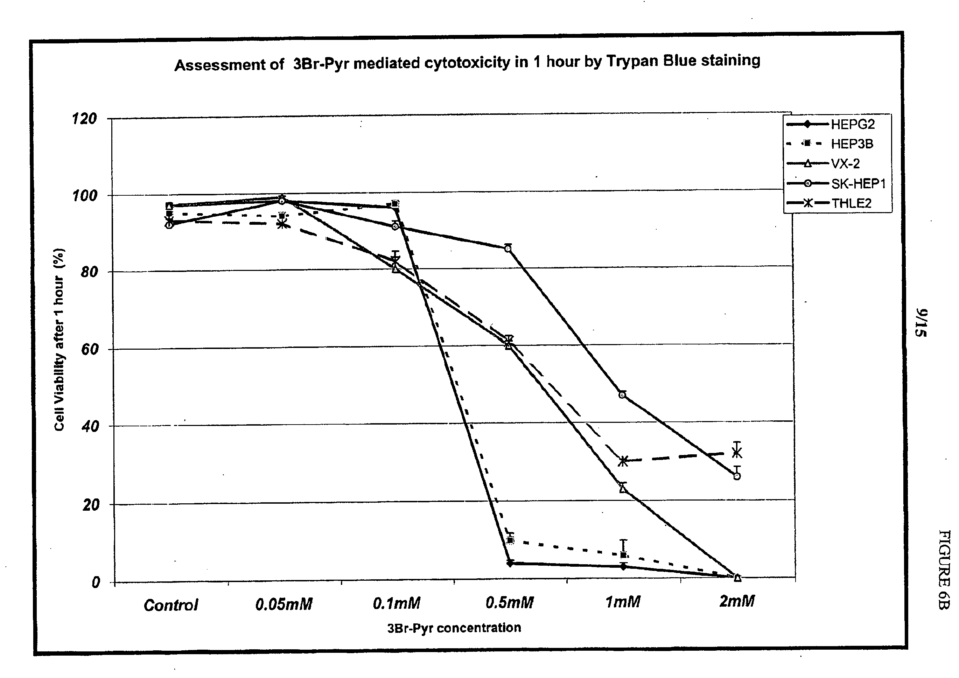

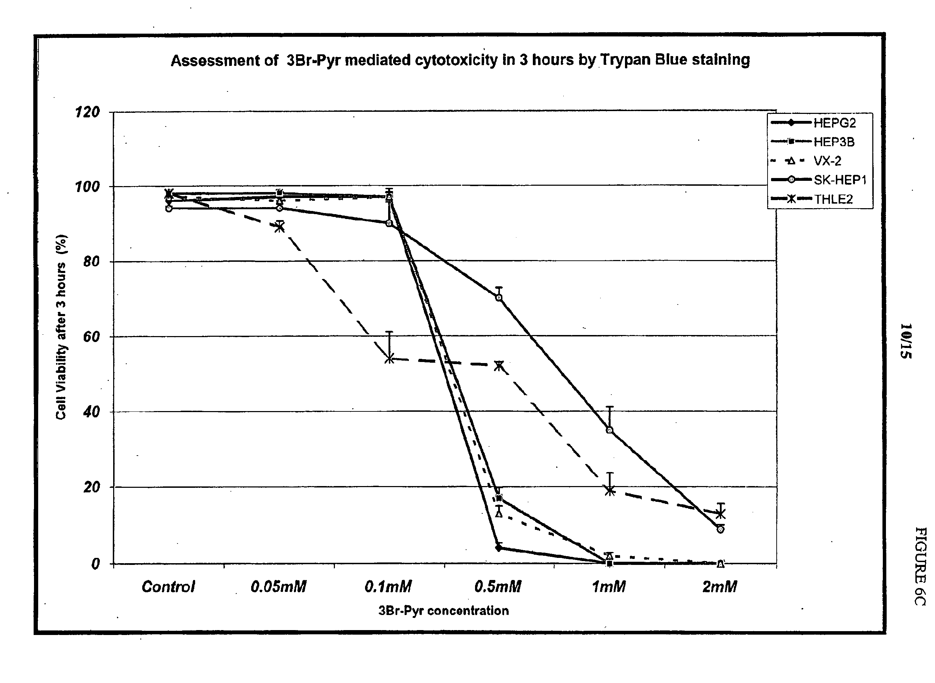

Caspase-3 activity and reduced cell viability was also measured in cell lines (HepG2, THLE-2, and VX2 cell lines) following 3-bromopyruvate treatment (1mM for 3 hour) (Figure 6A). Capsase-3 activity increased in each cell line following treatment and cell viability decreased following treatment. An increase in caspase-3 activity indicated the mechanism of cell death is apoptotic. Figures 6B and C show cell viability is reduced in each cell line with increasing 3-bromopyruvate concentration after 1 and 3 hours of treatment.

### Example 22: Specificity of 3-bromopyruvate as an anti-glycolytic agent in vivo.

After having established the anti-glycolytic effect of 3-bromopyruvate *in vitro,* we designed a series of experiments to confirm this property *in vivo.* For this purpose, two different animal models, the Lewis rat mammary tumor model and the orthotopic VX2 rabbit liver cancer model were used. Since glucose uptake can be monitored using fluorine-labeled deoxyglucose (FDG), potential sites of decreased FDG uptake as a result of 3-bromopyruvate activity could be identified. The effects of intraperitonealy (IP) injected 3-bromopyruvate in non-tumor bearing rats were compared to those in tumor bearing rats. The tumors were derived from the rat mammary tumor (RMT) cell line and transplanted into the back adipose tissue of the rat and allowed to grow for 10 days. After having determined the maximum tolerated dose, all animals received two bolus doses (one hour apart) of 3-bromopyruvate injected IP. The FDG was injected 30 minutes after the first injection of 3-bromopyruvate and all animals were sacrificed 60 minutes after the FDG was injected. The tumors and all major organs were removed, weighed and analyzed in a gamma counter. In the non-tumor bearing rats, injection of 3-bromopyruvate caused significant decrease in FDG uptake (10 fold decrease in percentage of injected dose per gram of tissue) only in the back adipose tissue (BAT), which is known to have a highly glycolytic phenotype and is believed to be involved in thermal regulation. 3-bromopyruvate affected no other organs. On the other hand, in the tumor-bearing rats, FDG uptake was significantly decreased (50% decrease) only in the tumor after IP injection of 3-bromopyruvate, confirming the notion of selectivity of 3-bromopyruvate. As with the non tumor bearing rats, all other organs were unaffected confirming the extraordinary, targeting ability of 3-bromopyruvate. Here, the tumor simply acted as a sump for 3-bromopyruvate. Thus, 3-bromopyruvate can be used *in vivo* as a potent anti-glycolytic agent to target cancer cells.

To develop a new therapeutic strategy to cure patients with hepatocellular carcinoma, a similar series of experiments was conducted on the orthotopic rabbit model of liver cancer (VX2). The effects of 3-bromopyruvate injected intraarterially (IA) was compared to those of 3-bromopyruvate injected intravenously (IV) on FDG uptake in the VX2 tumor. The optimal dose of 3-bromopyruvate for both IA and IV administration was then determined through an extensive dose-response analysis. In the IA group, 3-bromopyruvate was infused over one hour, FDG injected at the 30-minute mark and all animals were then sacrificed one hour after the FDG injection. Tumors and all major organs were removed, weighed and analyzed in a gamma counter. Significantly, in the IA group, FDG uptake was 80% reduced in the tumor. No difference in uptake was identified in any other organs. In contrast, in the IV group, FDG uptake was reduced (30% decrease), but not inhibited in the tumor and decreases in FDG uptake were also recorded in the lung, brain and liver. These results confirmed the potent anti-glycolytic effects of 3-bromopyruvate, and provided justification for administering 3-bromopyruvate intraarterially directly to the tumor.

### Example 23: In-vitro analysis of specific binding of 3-bromopyruvate to Type ll hexokinase in HCC cell line HepG2.

In a further study, the human hepatocellular carcinoma cell line (HCC), Hep G2, was treated with ¹⁴C-labeled 3-bromopyruvate for a period of 1 hour in HBSS. The cells were harvested, total protein extracted, purified and used to run a western blot. They were then transferred onto a membrane, probed with a Type II hexokinase specific antibody and the antibody binding signal was captured on film through Chemiluminescent detection system (western blotting). The corresponding gel was also separately exposed to an autoradiograph. The signal produced by the Type II hexokinase antibody (on the western blot membrane) exactly matched with that of the ¹⁴C-3-bromopyruvate (on the autoradiograph) showing that 3-bromopyruvate directly binds Type II hexokinase. This demonstrates the potential of 3-bromopyruvate as a new potent anti-cancer agent that functions through the inhibition of Type II hexokinase and therein tumor glycolysis.

### Example 24: Determination of optimal dose, tumor response, toxicity and delay of tumor progression.

In a longitudinal study to investigate the effect of 3-bromopyruvate treatment in the VX2 rabbit model, it was observed that although the treated rabbits lived longer than the untreated ones (statistically significant), the treated rabbits were not cured of their tumors and died within weeks of treatment of massive tumor growth. The extremely aggressive nature of the VX2 tumor was not stopped by the treatment and the tumor re-grew despite the high initial percentage of cancer cell death. Further studies were thus needed to determine the optimal dose and method of infusion of 3-bromopyruvate to attain significant long-term survival, complete eradication of the tumor.

In order to determine the therapeutic dose of 3-bromopyruvate for intravenous (IV) administration, a dose-escalation study was conducted in normal Lewis rats (non-tumor bearing). Each group of animals (6 rats per group) received incremental doses of 3-bromopyruvate intravenously (2.5mL of 5, 10, 15, 20, 25 and 50mM 3-bromopyruvate). The animals were observed for a period of 3 weeks and watched for any evidence of toxicity. The therapeutic dose was found to be 20mM. At the highest dose of 50mM, all the animals in the group died within 15 minutes. It was observed that all of the organs were found to be normal by histopathologic analysis and that there was no evidence of corrosive effects of 3-bromopyruvate even at that high dose. All doses lower than 20 mM were well-tolerated and caused no deaths. In addition, there was no evidence of clinical distress (lack of appetite, urination, or significant change in body weight). Analysis of major organs (brain, lung, heart, liver, kidney, spleen, stomach, G.I, bladder and skeletal muscle) at necropsy confirmed the safety of these doses, as all were without any damage. These results therefore established the therapeutic dose of 3-bromopyruvate to be 20mM or 16.8 mg/kg.

In addition, an acute toxicity study was performed to determine the effects of 3-bromopyruvate administered IV. Based on the data acquired in rats, rabbits (2 animals per group followed for 1 week) were given 25 ml of 15, 20, and 25 mM 3-bromopyruvate as a single IV dose (i.e., using dose identified for rats). Results of the rabbit experiment paralleled those of the rats with 20mM or 16.8 mg/kg 3-bromopyruvate calculated to be the therapeutic dose, with no evidence of any organ toxicity.

In an earlier study, 0.5mM 3-bromopyruvate administered intraarterially was demonstrated to effectively kill most of the tumor cells, whereas the same dose delivered IV had no effect on the tumor. In a therapeutic dose study, 25 ml of 2.5 and 5mM 3-BrPA were injected as a single intraarterial bolus over two minutes. Although these doses (2.5 and 5 mM) were 0.5 and 1 log higher than the 0.5mM dose previously investigated, these doses were well within the IV therapeutic dose established in rats and rabbits. It was observed that the 5mM concentration of 3-bromopyruvate completely destroyed the tumor, but also caused widespread necrosis of the surrounding healthy liver resulting in the death of all the rabbits within the group. However, all of the other major organs were found to be completely uneffected on histopathological analysis at necropsy, indicating a predominantly loco-regional toxicity of 3-bromopyruvate when delivered intraarterially. The lower dose of 2.5mM also achieved complete tumor destruction, but the surrounding healthy liver still showed large areas of necrosis and fatty changes making the animals extremely sick and barely able to survive for more than 48 hours after treatment.

Since patients with HCC also suffer from underlying liver disease such as cirrhosis and hepatitis, an important translational consideration for therapy is the absolute necessity to protect and preserve healthy liver parenchyma. To lessen the degree of liver toxicity, different temporal modalities of intraarterial injection were tested. One possibility was that such a large volume, delivered within the short time of 2 minutes may have added physical and chemical toxic effects on the liver. Thus, a 1-hour continuous infusion and a serial infusion of 2 boluses administered an hour apart were compared against the original single bolus dose. The volume of 25mL and the concentration of 2.5 mM 3-bromopyruvate were kept constant. Remarkably, liver toxicity was considerably less in the 1-hour continuous infusion group than in either of the other two modalities. In each of these experiments, rabbits were sacrificed 48 hours after treatment and representative sections obtained of tumor and normal tissues (7 slides/sample). The samples were subjected to histopathological analysis to determine percentage tumor cell death and damage to the surrounding normal liver tissue. This was then confirmed with the TUNEL assay (Figure 7) and Ki-67 immunohistochemistry. The results clearly demonstrated a significant advantage in using a 1-hour continuous infusion as the temporal modality of choice in the intrraarterial injection of 3-bromopyruvate for the treatment of rabbit VX2 liver cancer. The prolonged slow infusion eliminated most, if not all, of the unwanted damage to the normal liver enabling the use of a much higher dose of 3-bromopyruvate to ensure complete destruction of the tumor.

Having determined that prolonged intraarterial infusion of 3-bromopyruvate was the optimal method of administration, a dose-response analysis was conducted. In part, this dose-response analysis was prompted by the persistent toxicity to healthy liver encountered at 2.5 mM. The objective was to optimize the 1-hour infusion in order to achieve complete tumor eradication without any toxicity to the normal liver parenchyma. Rabbits were intraarterially infused over 1-hour with doses ranging between 0.5 and 2.5 mM in 0.25 mM increments. The rabbits were fasted 24 hours prior to treatment and euthanized 48 hours after treatment. Rabbits treated with 25mL of 1.75 mM 3-bromopyruvate had 100% tumor cell destruction without any damage to the normal liver. This total destruction was identified by histopathology (H&E) and confirmed by the TUNEL assay (Figure 7). Higher doses of 3- bromopyruvate increased toxicity to the healthy liver tissue. One possible explanation for this finding may be that 3-bromopyruvate at higher doses may have other cellular and chemical functions that are much more toxic to the normal liver parenchyma therein impeding the delivery of 3-bromopyruvate exclusively to the tumor. Thus, the healthy liver's ability to metabolize 3-bromopyruvate was optimal at the concentration of 1.75 mM.

To further explore use of the continuous infusion method of delivery, the effect of a 2-hour continuous infusion of 50mL 1.75 mM 3-BrPA was tested. Although the amount of 3-bromopyruvate was doubled, there was no significant difference in tumor kill or in liver toxicity demonstrating that both the infusion time as well as concentration played a role in the determination of the optimal therapeutic dose. Thus, a 1-hour intraarterial infusion of 25mL of 1.75 mM 3-bromopyruvate was identified as an optimal dose since it caused complete destruction of VX2 rabbit liver tumors (as seen on histopathology) without any evidence of major organ toxicity.

VX2 tumors are extremely aggressive and are rendered more aggressive and difficult to treat by the passage of this tumor from generation to generation (>100) in the thigh of a rabbit making each generation more virulent than the last. In early longitudinal survival studies using the VX2 tumor rabbit model, animals with 100% tumor kill showed a 75% delay of tumor progression when compared with control animals treated with only saline (IA). As described herein continuous intraarterial infusion of 1.7 mM 3-bromopyruvate is an effective and potent therapy for treating liver cancer.

### Example 25: Utility of PET/CT imaging to assess tumor response after therapy with 3-bromopyruvate.

To further confirm the tumor destruction using 3-bromopyruvate, Positron Emission Tomography (PET)/CT imaging was conducted in rabbits before and after a single 1-hour intraarterial (infusion) therapy with 3-bromopyruvate. A complete disappearance of tumor activity on the post-treatment scans was observed only 2 hours after treatment. Since 3-bromopyruvate acts extremely quickly, PET/CT images were obtained within 2 hours of therapy to examine the process of making a decision regarding immediate re-treatment. After the PET/CT images were obtained, the 2 rabbits were sacrificed, their liver explanted and submitted for histopathologic analysis. Various staining methods that the tumor was completely (100%) dead and that the mechanism of cell death was apoptotic, thus confirming the use of PET imaging in assessing tumor response.

### Example 26: Determination of the temporal biodistribution profile of 3-bromopyruvate in an animal model of liver cancer.

In order to properly design a treatment protocol that would ensure survival of the animal with the least amount of toxicity, the biodistribution of 3-bromopyruvate given either IV or intraarterially over a period of 24 hours was investigated. For this series of experiments, radioactive 3-bromopyruvate using ¹⁴C was synthesized. The use of ¹⁴C labeled 3-bromopyruvate would allow monitoring in each tissue.

### Synthesis of radiolabeled (1-¹⁴C) 3 bromopyruvate.

After screening various radioactive markers, carbon 14 was selected as the radiotracer for labeling of 3-bromopyruvate because of its relative ease of synthesis and long half-life. The labeling process consisted of incorporating carbon 14 into pyruvic acid at the first carbon atom resulting in (1-¹⁴C) pyruvic acid, which was then reacted with elemental bromine leading to the synthesis of (1-¹⁴C) 3-bromopyruvate. The resulting product was purified by preparative HPLC and its radiochemical purity was determined by analytical HPLC.

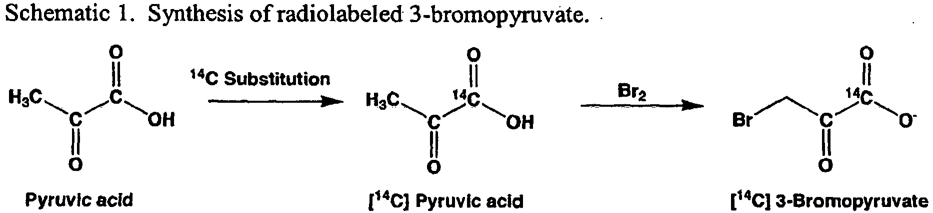

### Analysis of the biodistribution and pharmacokinetics in vivo of 3-bromopyruvate using¹⁴C radiolabeling in the rabbit Vx-2 model of liver cancer.

36 rabbits were divided into two groups of 18 animals each. Group 1 was treated intraarterially and group 2 received intravenous injection of 3-bromopyruvate. Each group was further subdivided into six groups of three rabbits each, corresponding to various time points of sacrifice (0, 30, 60, 120 and 240 minutes and 24 hours). All rabbits were fasted for 24 hours prior to treatment. The rabbits treated intraarterially received a one-hour infusion of 25cc of 1.75mM 3-bromopyruvate in PBS and those treated intravenously received a one-hour infusion of 25cc of 20mM 3-bromopyruvate in PBS. The difference in doses of 3-bromopyruvate between intraarterial and intravenous administration is based on the optimal dose for each method of administration. Radiolabeled 3-bromopyruvate (200 µCi, 100 µL) was added to each 25ml treatment solution. One hour before sacrifice, 1ml of 18FDG (1mCi) was injected via an ear vein to monitor glucose uptake. In comparison to the IV injection, it was observed that when given intraarterially, 3-bromopyruvate concentrated in the tumor immediately (at time 0) and stayed within the tumor up to 24 hours after injection (Figure 8), albeit in lower concentration. The clearance of 3-bromopyruvate was accomplished mostly through the liver without any associated tissue damage and also to a lesser extent through the kidneys. There was no accumulation of the drug in any other organs including brain, lungs and heart. When 3-bromopyruvate was injected IV, there was no significant uptake by any organs (heart, lung and brain), however, the IV dose was10 times greater than the dose given intraarterially. In the brain, an organ with a naturally high metabolic activity, levels of 3-bromopyruvate were virtually undetectable (Figure 8), demonstrating that the drug does not cross the blood brain barrier. In addition, there was no inhibition of FDG (glucose) uptake in any of the organs demonstrating that 3-bromopyruvate did not affect glucose uptake by these organs. At histopathologic analysis, none of the organs showed any associated tissue damage from 3-bromopyruvate.

The purpose of the injection of FDG was to correlate the effects of 3-bromopyruvate with glucose uptake. It was observed that the tumor showed significant uptake of 3-bromopyruvate (maximal at 4 hours after IV injection) and concomitant inhibition of FDG uptake, confirming the principle that 3-bromopyruvate targets the tumor, which acts as a sump, and shuts down tumor metabolism. This finding, also present when the drug was given intraarterially, was more pronounced and the accumulation of 3-bromopyruvate is prolonged compared to IV administration.

### Example 27: Survival analysis comparing 3-bromopyruvate treatment and control group

The control group consisted of 10 animals and the treatment group consisted of 11 animals. The Kaplan-Meier method was used to estimate survival curves. As shown in Figure 9, the treatment group is associated with longer survival as compared to the control group. As each rabbit experienced an event, the equal mean event times between the control and treatment groups was tested based on a t-test and rejected at the 0.05 level of significance, where the control group significantly differed on average, as compared to the treatment group (p-value< 0.001, 95% C.I for mean difference of treatment - control, (27.36, 35.44)). The mean survival time in the control group is 18.6 days as compared to the treatment group, with 50.0 days.

### Example 28: Preparation of 3-bromopyruvate loaded liposomes using reverse phase evaporation method and cell toxicity of 3-bromopyruvated liposomes to liver carcinoma cells (Huh7)

3BrPA liposome showed close to 100% killing to Huh7 cells, while blank liposome showed limited (<10% at low concentration) cell toxicity to Huh7 at similar lipid concentration (Figure 10). 3BrPA liposomes did not seem to be active after incubating at 4°C for 4-6 days, which suggests the liposome might be leaky. This is very likely related to the composition of the lipids which have low transition temperatures. Low transition temperature allows the fabrication to be carried on at low temperature, which helps maintain the activity of 3BrPA. But low transition temperature may also lead to the instability and leakage of liposomes.

The concentration of 3BrPA in liposome is estimated based on the assumption that 10% of the drug was encapsulated. The blank liposome control is assumed to have the same concentration of lipids as 3BrPA lioposome.

### List of citations

U.S. Patent Applications 2003/0087961 and 2007/0203074.
J. Barnard, et al. (1993) J. Biol. Chem. 268:3654-3661. Sharma, et al. (2000) Nature Str. Biol. 8:663-668. M.C.Kew, et al. (1998) In: Feldman, M., et al (eds.) Gastrointestinal and Liver Disease:Pathophysiology, Diagnosis, and Management, pub. W.B.Saunders Co.: 1404-1415. P. Rous, et al. (1952) J. Exp. Med. 96:159-174. S.Pauser, et al. (1996) Cancer Res. 56:1863-1867. J.-F. Geschwind, et al. (2000) JVIR 11:1245-1255. M.Sakurai, et al. (1984) Cancer 54:387-392. M. Soulen, (1994) Oncology 8:77-84. S. Weinhouse, (1972) Cancer Res. 32:2007-2016. P.L.Pedersen, (1978) Prog. Exp. Tumor Res. 22:190-274. E. Bustamante, et al. (1981) J. Biol. Chem. 256:8699-8704. D.M. Parry, et al. (1983) J. Biol. Chem. 258:10904-10912. Y.Shinohara, et al. (1991) FEBS Lett. 291:55-57. A. Rempel, et al. (1996) Cancer Res. 56:2468-2471. S.P. Mathupala, et al. (1995) J. Biol. Chem. 270:16918-16925. S. Pilkis, et al. (1994) J. Biol. Chem. 269:21925-21928. R. A. Harris, (1997) In: T.M.Devlin, (ed.) Textbook of Biochemistry with Clinical Correlations, pub. Wiley-Liss: 267-359. K.K.Arora, et al. (1988) J. Biol. Chem. 263:14422-14428. E.F. Greiner, et al. (1994) J. Biol.Chem. 269:31487-31490. A.Rempel, et al. (1998)Cell Growth and Oncogenesis :3-14. N.Oya, et al. (1993) J. Nuclear Med. 34:2124-2129. Liang, T.J., et al. (1993) Hepatology. 18:1326-1333. El-Serag, H.B. (2001) Clin. Liver Dis. 5:87-107. El-Serag, H.B., et al. (1999) New Engl. J. Med. 340:745-750. Saha, S., et al. (2001) Science 294:1343-1346. Geschwind, J.F., et al. (2000) J. Vasc. Interv. Radiol. 11:1245-1255. Venook, A., (1994) J. Clin. Oncol. 12:1323-1334. Levin, B., et al. (1995) N. Engl. J. Med., 332:1294-1296. Seong, J., et al. (1999) Intl. J. Rad. Oncol. Biol. Phys. 43:393-397. Breedis, C., et al. (1954) Am. J. Pathol. 30:969- 985. Pedersen, P. L. (1999) J. Bioenerg. Biomemb. 31:291-304. Ko, Y.H., et al. (1990) Arch. Biochem. Biophys. 278:373-380. Ko, Y.H., et al. (2001) Cancer Lett., 173:83-91. Rous, P., et al. (1952) J. Exp. Med. 96:159-174. Pauser, S., et al. (1996) Cancer Res. 56:1863-1867. Weinhouse, S. (1972) G.H.A. Clowes Memorial Lecture. Cancer Res. 32:2007-2016. Arora, K.K., et al. (1988) J. Biol. Chem. 263:14422-14428. Meister, A., et al. (1983) Ann. Rev. Biochem. 52:711-760. Deneke, S.M., et al. (1989) Am. J. Physiol. 257:L163-L173. Sakurai, M., et al. (1984) Cancer, 54:387-392,1984. Soulen, M. (1994) Oncology, 8:77-84, 1994. US Patent Application 20020068711. Shin, SW., et al. (2006) Act a Radiologica, 47: 1036 - 1041 , 2006.

## Claims

1. An effective amount of 3-bromopyruvate for use in treating cancer in a subject in need thereof, wherein the effective amount of 3-bromopyruvate is administered as a continuous intraarterial infusion to the subject from one hour to four hours.

2. An effective amount of 3-bromopyruvate for use of claim 1, wherein the continuous intraarterial infusion is administered to the subject from 1 to 3 hours.

3. An effective amount of 3-bromopyruvate for use of claim 1, wherein the effective amount of 3-bromopyruvate is a dose of 3-bromopyruvate between the range of 0.5 to 2.5 mM.

4. An effective amount of 3-bromopyruvate for use of claim 1, wherein the effective amount of 3-bromopyruvate is a dose of 1 to 2 mg/kg.

5. An effective amount of 3-bromopyruvate for use of claim 1, further comprising administering a second agent.

6. An effective amount of 3-bromopyruvate for use of claim 5, wherein the second agent is selected from the group consisting of: a chemotherapeutic agent, a scavenger compound, and radiation.

7. An effective amount of 3-bromopyruvate for use of claim 5, wherein the second agent is one or more chemotherapeutic agent.

8. An effective amount of 3-bromopyruvate for use of claim 7, wherein the one or more chemotherapeutic agent is selected from the group consisting of: altretamine, asparaginase, BCG, bleomycin sulfate, busulfan, carboplatin, carmusine, chlorambucil, cisplatin, claladribine, 2-chlorodeoxyadenosine, cyclophosphamide, cytarabine, dacarbazine imidazole carboxamide, dactinomycin, daunorubicin - dunomycin, dexamethosone, doxurubicin, etoposide, floxuridine, fluorouracil, fluoxymesterone, flutamide, fludarabine, goserelin, hydroxyurea, idarubicin HCL, ifosfamide, interferon alfa,
interferon alfa 2a, interferon alfa 2b, interfereon alfa n3, irinotecan, leucovorin calcium, leuprolide, levamisole, lomustine, megestrol, melphalan, L-sarcosylin, melphalan hydrochloride, MESNA, mechlorethamine, methotrexate, mitomycin, mitoxantrone, mercaptopurine, paclitaxel, plicamycin, prednisone, procarbazine, streptozocin, tamoxifen, 6-thioguanine, thiotepa, vinblastine, vincristine and vinorelbine tartrate.

9. An effective amount of 3-bromopyruvate for use of claim 6, wherein the scavenger compound is selected from the group consisting of: lipoleic acid, glutathione and cysteine.

10. An effective amount of 3-bromopyruvate for use of claim 1, wherein the continuous intraarterial infusion is administered to the subject for 1 hour.

## Patentansprüche

1. Wirksame Menge von 3-Brompyruvat zur Verwendung für die Behandlung von Krebs bei einem Subjekt, das deren bedarf, wobei die wirksame Menge von 3-Brompyruvat dem Subjekt 1 bis 4 Stunden als kontinuierliche intraarterielle Infusion verabreicht wird.

2. Wirksame Menge von 3-Brompyruvat zur Verwendung nach Anspruch 1, wobei die kontinuierliche intraarterielle Infusion dem Subjekt etwa 1 bis 3 Stunden verabreicht wird.

3. Wirksame Menge von 3-Brompyruvat zur Verwendung nach Anspruch 1, wobei die wirksame Menge von 3-Brompyruvat eine Dosis von 3-Brompyruvat im Bereich von 0,5 bis 2,5 mM ist.

4. Wirksame Menge von 3-Brompyruvat zur Verwendung nach Anspruch 1, wobei die wirksame Menge von 3-Brompyruvat eine Dosis von 1 bis 2 mg/kg ist.

5. Wirksame Menge von 3-Brompyruvat zur Verwendung nach Anspruch 1, ferner umfassend die Verabreichung eines zweiten Agens.

6. Wirksame Menge von 3-Brompyruvat zur Verwendung nach Anspruch 5, wobei das zweite Agens aus der Gruppe ausgewählt ist, die aus einem chemotherapeutischen Agens, einer Scavenger-Verbindung und Bestrahlung besteht.

7. Wirksame Menge von 3-Brompyruvat zur Verwendung nach Anspruch 5, wobei es sich bei dem zweiten Agens um ein chemotherapeutisches Agens oder mehrere chemotherapeutische Agenzien handelt.

8. Wirksame Menge von 3-Brompyruvat zur Verwendung nach Anspruch 7, wobei das eine chemotherapeutische Agens oder die mehreren chemotherapeutischen Agenzien ausgewählt ist/sind aus der Gruppe: Altretamin, Asparaginase, BCG, Bleomycinsulfat, Busulfan, Carboplatin, Carmustin, Chlorambucil, Cisplatin, Cladribin, 2-Chlor-2'-desoxyadenosin, Cyclophosphamid, Cytarabin, Dacarbazin-Imidazolcarboxamid, Dactinomycin, Daunorubicin/Daunomycin, Dexamethason, Doxorubicin, Etoposid, Floxuridin, Fluoruracil, Fluoxymesteron, Flutamid, Fludarabin, Goserelin, Hydroxyharnstoff, Idarubicin-HCL, Ifosfamid, Interferon-alpha, Interferon-alpha-2a, Interferon-alpha-2b, Interferon-alpha-n3, Irinotecan, Calciumfolinat, Leuprolid, Levamisol, Lomustin, Megestrol, Melphalan, Sarkosyl L, Melphalanhydrochlorid, MESNa, Mechlorethamin, Methotrexat, Mitomycin, Mitoxantron, Mercaptopurin, Paclitaxel, Plicamycin, Prednison, Procarbazin, Streptozocin, Tamoxifen, 6-Thioguanin, Thiotepa, Vinblastin, Vincristin und Vinorelbintartrat.

9. Wirksame Menge von 3-Brompyruvat zur Verwendung nach Anspruch 6, wobei die Scavenger-Verbindung aus der Gruppe ausgewählt ist, die aus Liponsäure, Glutathion und Cystein besteht.

10. Wirksame Menge von 3-Brompyruvat zur Verwendung nach Anspruch 1, wobei die kontinuierliche intraarterielle Infusion dem Subjekt 1 Stunde lang verabreicht wird.

## Revendications

1. Quantité efficace de 3-bromopyruvate pour l'utilisation dans le traitement du cancer dans un sujet qui en a besoin, la quantité efficace de 3-bromopyruvate étant administrée audit sujet par infusion intraartérielle continue pendant une à quatre heures.

2. Quantité efficace de 3-bromopyruvate pour l'utilisation selon la revendication 1, l'infusion intraartérielle continue étant administrée au sujet pendant environ 1 à 3 heures.

3. Quantité efficace de 3-bromopyruvate pour l'utilisation selon la revendication 1, la quantité efficace du 3-bromopyruvate étant un dosage de 3-bromopyruvate compris entre 0,5 et 2,5 mM.

4. Quantité efficace de 3-bromopyruvate pour l'utilisation selon la revendication 1, la quantité efficace du 3-bromopyruvate étant un dosage de 1 à 2 mg/kg.

5. Quantité efficace de 3-bromopyruvate pour l'utilisation selon la revendication 1, comportant en outre l'administration d'un deuxième agent.

6. Quantité efficace de 3-bromopyruvate pour l'utilisation selon la revendication 5, ledit deuxième agent étant choisi dans le groupe constitué par un agent chimiothérapeutique, un nécrophage et de la radiation.

7. Quantité efficace de 3-bromopyruvate pour l'utilisation selon la revendication 5, ledit deuxième agent étant un ou plusieurs agent(s) chimiothérapeutique(s).

8. Quantité efficace de 3-bromopyruvate pour l'utilisation selon la revendication 7, l'un ou les plusieurs agent(s) chimiothérapeutique(s) étant choisi(s) dans le groupe constitué par l'altrétamine, l'asparaginase, le BCG, le sulfate de bléomycine, le busulfan, le carboplatine, le carmustin, le chlorambucil, le cisplatine, le cladribine, le 2-chlorodésoxyadénosine, le cyclophosphamide, la cytarabine, la dacarbazine du carboxamide d'imidazol, la dactinomycine, la daunorubicine/daunomycine, la dexaméthasone, la doxorubicine, l'étoposide, la floxuridine, le fluorouracile, la fluoxymestérone, le flutamide, la fludarabine, la goséréline, l'hydroxyurée, le chlorhydrate d'idarubicine, l'ifosfamide, l'interféron alpha, l'interféron alpha 2a, l'interféron alpha 2b, l'interféron alpha n3, l'irinotécan, la leucovorine calcium, la leuprolide, le lévamisole, le lomustine, le mégestrol, le melphalane, le L-sarcosyline, le chlorhydrate de melphalane, le MESNa, la méchloréthamine, la méthotrexate, la mitomycine, la mitoxantrone, la mercaptopurine, le paclitaxel, la plicamycine, la prednisone, la procarbazine, la streptozocine, la tamoxifène, la 6-thioguanine, le thiotépa, le vinblastine, le vincristine et le tartrate de vinorelbine.

9. Quantité efficace de 3-bromopyruvate pour l'utilisation selon la revendication 6, ledit nécrophage étant choisi dans le groupe constitué par l'acide lipoïque, la glutathione et la cystéine.

10. Quantité efficace de 3-bromopyruvate pour l'utilisation selon la revendication 1, l'infusion intraartérielle continue étant administrée au sujet pendant 1 heure.
